# EUROPEAN PATENT APPLICATION

(11) **EP 3 892 096 A1**
(43) Date of publication of application: **13.10.2021**
(21) Application number: 20210139.0
(22) Date of filing: 16.03.2012
(51) Int. Cl.: A01N 63/20, A01N 63/22, A01N 63/25, A01N 63/27, A01H 3/00

(54) **SCREENING METHODS**

(30) Priority: 17.03.2011 NZ 11588048
(62) Divisional of application: 12757224.6
(71) Applicant: BioConsortia, Inc., Davis, CA 95618 (US)
(72) Inventor: WIGLEY, Peter, Auckland 1052 (NZ); GEORGE, Caroline Elizabeth, Auckland 1072 (NZ)
(74) Representative: HGF

(57) **Abstract**

The present invention relates to methods for the screening, identification and/or application of microorganisms and/or compositions of use in imparting beneficial properties to plants, and microorganisms and compositions identified therefrom.

## Description

### FIELD

The present invention relates to methods for the screening, identification and/or application of microorganisms of use in imparting beneficial properties to plants.

### BACKGROUND

Geography, environmental conditions, disease and attack by insects are major factors influencing the ability to viably grow and cultivate different species of plant. Such factors can have a significant downstream economic and social impact on communities around the world. There would be benefit in identifying products and methods which might impart beneficial properties to a plant species to allow it to grow in a variety of geographical locations, in different weather conditions, to survive disease and to be resistant to attack by insects, for example.

Selective breeding techniques have been used to this end. Selective breeding relies principally on genetic diversity in a starting population coupled with selection to achieve a plant cultivar with characteristics beneficial for human use. As the available, unused genetic diversity of cultivatable plant species has diminished, the potential for improvement has decreased. This situation has stimulated the growth of plant genetic modification in which genes from closely related species are introgressed into the new cultivar to provide a new genetic base for imparting desirable traits into new cultivars. However, this process is extremely costly, slow, limited in its scope and fraught with regulatory difficulties. Few commercial successes have eventuated from over two decades of large-scale investment into this technology.

Despite many decades of successful scientific research into the conventional breeding of highly-productive crops and into development of transgenic crops, relatively little research effort has been directed at development of improved plant growth and survival via other means.

The importance of providing "good" soil with a rich microbial diversity via composts, complex biomaterial fertilisers e.g. blood and bone, to plants to ensure their healthy growth has been understood by home gardeners' and producers of organic foods. However, the inventors have recognised that the complexity of the plant-microorganism associations that underpin the observable benefits is poorly understood. Benefits to plant growth and health in such soils are often microbially-mediated through improved nutrient availability. This may be the result of solubilisation of minerals from the soil biomass itself, or from colonization of the plants with microorganisms in endophytic, epiphytic or rhizospheric associations leading to nitrogen fixation, resistance to pests and diseases through direct microbial competition within the plant, or the elicitation of plant defence reactions. The science community has produced literature on the diverse mechanisms of endophytic, epiphytic and rhizospheric plant microorganism associations, largely in relation to crop plants and their soils. The nature of some associations is known, encompassing the genetic basis of plant-induced metabolite production by specific organisms and the reverse influence of the microbe on gene expression in the plant (e.g. *Neotyphodium* spp), and increases in plant growth following microbial application to certain crop plants or seeds has been documented. However, despite the potential of microorganisms to improve plant growth, commercial success is limited to a relatively small range of specific microbial applications e.g. *Rhizobium* spp. to legume seeds, or the use of products resulting from "uncontrolled" microbial fermentations e.g. compost teas, seaweed fermentations, fish waste fermentations etc.

There are many specific strains of potentially beneficial microorganisms for association with specific plant cultivars, making the task of finding an appropriate strain(s) for any particular crop a very onerous procedure. Current means primarily focus on the application of microorganisms singly or in limited combinations. Such microorganisms are likely to have been selected for specific potential properties based on their identity. It would be useful if there was no requirement for knowledge of microbial identity for success.

Bibliographic details of the publications referred to herein are collected at the end of the description.

### OBJECT

It is an object of the present invention to provide a method for the selection of one or more microorganism which is of use in imparting beneficial properties to a plant which overcomes or ameliorates at least one of the disadvantages of known methods.

Alternatively it is an object of the invention to provide a method and/or system for assisting in the improvement of one or more plants.

Alternatively, it is an object to at least provide the public with a useful choice.

### STATEMENT OF INVENTION

In a first broad aspect of the present invention there is provided a method for the selection of one or more microorganisms capable of imparting one or more beneficial property to a plant, the method comprising at least the steps of:
a) subjecting one or more plant (including seeds, seedlings, cuttings, and/or propagules thereof) to a growth medium in the presence of one or more microorganisms;
b) applying one or more selective pressure during step a);
c) selecting one or more plant following step b); and,
d) isolating one or more microorganisms from said one or more plant selected in step c).

In one embodiment, the one or more microorganisms are selected from the microorganisms detailed herein after.

In one embodiment, the growth medium is selected from the growth media detailed herein after.

In one embodiment, the step of subjecting one or more plant to a growth medium involves growing or multiplying the plant.

In one embodiment, one selective pressure is applied in step b).

In one embodiment, the selective pressure is biotic and includes but is not limited to exposure to one or more organisms that are detrimental to the plant. In one embodiment, the organisms include fungi, bacteria, viruses, insects, mites and nematodes.

In another embodiment, the selective pressure is abiotic. Abiotic selective pressures include, but are not limited to, exposure to or changes in the level of salt concentration, temperature, pH, water, minerals, organic nutrients, inorganic nutrients, organic toxins, inorganic toxins, and metals.

In one embodiment, the selective pressure is applied during substantially the whole time during which the one or more plant is subjected to the growth medium and one or more microorganisms. In one embodiment, the selective pressure is applied during substantially the whole growth period of the one or more plant. Alternatively, the selective pressure is applied at a discrete time point.

In one embodiment, the one or more plant is selected on the basis of one or more phenotypic trait. In one preferred embodiment, the one or more plant is selected based on the presence of a desirable phenotypic trait. In one embodiment, the phenotypic trait is one of those detailed herein after.

In one embodiment, the one or more microorganisms are isolated from the root, stem and/or foliar (including reproductive) tissue of the one or more plants selected. Alternatively, the one or more microorganisms are isolated from whole plant tissue of the one or more plants selected.

In one embodiment, the one or more microorganisms are isolated from the one or more plants any time after germination.

In one embodiment, where two or more microorganisms are isolated in step d), the method further comprises the steps of separating the two or more microorganisms into individual isolates, selecting two or more individual isolates, and then combining the selected two or more isolates.

In another embodiment, the method further comprises repeating steps a) to d) one or more times, wherein the one or more microorganisms isolated in step d) are used in step a) of the successive repeat.

In another embodiment, the method further comprises repeating steps a) to d) one or more times, wherein where two or more microorganisms are isolated in step d), the two or more microorganisms are separated into individual isolates, two or more individual isolates are selected and then combined, and the combined isolates are used in step a) of the successive repeat. Accordingly, where reference is made to using the one or more microorganisms isolated in step d) in step a) of the method, it should be taken to include using the combined isolates of this embodiment of the invention.

In one embodiment, one or more selective pressure applied in successive repeats of steps a) to d) is different. In another embodiment, the selective pressure(s) applied in successive repeats of steps a) to d) is the same.

In one embodiment, the method further comprises the following step which is conducted prior to step a): subjecting the one or more plant (including seeds, seedlings, cuttings, and/or propagules thereof) to a growth medium in the presence of one or more microorganisms, and after a desired period, isolating one or more microorganisms from said one or more plant. In a preferred embodiment, the one or more microorganisms isolated from the one or more plant, are used in step a) of the process. In one embodiment, this step may be conducted two or more times prior to step a).

In another embodiment of the invention, where one or more microorgansim(s) forms an association with a plant that allows vertical transmission from one generation or propagule to the next, step d) may be absent from the method or substituted by the step of multiplying the selected plant(s) from step c). Accordingly, the invention also provides methods for the selection of one or more plant harbouring one or more microorganisms capable of imparting one or more beneficial property to the one or more plants. The invention also provides plants selected by such methods.

In another embodiment, two or more methods of the invention may be performed separately and the one or more microorganisms isolated in step d) of each separate method combined. In one embodiment, the combined microorganisms are used in step a) when performing a further method of the invention.

In a second broad aspect, there is provided a method for assisting in the improvement of one or more plants, comprising arranging for the evaluation of said plant(s) in the presence of one or more microorganisms and/or compositions.

According to one embodiment, the plant(s) are for growing in a first region. The microorganism(s) may or may not (or at least to a significant extent) be present in the first region.

"Region" and "first region" are to be interpreted broadly as meaning one or more areas of land. The land areas may be defined by geographical / political / private land boundaries or by land areas having similar properties such as climate, soil properties, presence of a particular pest etc.

Preferably, the evaluation is performed in a second region in which the microorganism(s) are present, but this is in no way essential. Microorganisms may be obtained from other sources including microorganism depositaries and artificially associated with plant material and/or soil. Furthermore, while plant(s) may be cultivated in essentially a conventional manner but in a region having microorganisms not normally associated with the plant(s), at least in the first region, artificial growing environments may alternatively be used as would be appreciated by those skilled in the art. Thus, possible beneficial microorganism/plant relationships may be identified that would not necessarily normally be utilised.

Preferably, the step of arranging comprises arranging for one or more of:
- receipt or transmission of an identity of one or more plants or plant types to be evaluated;
- receipt or transmission of plant material from one or more plants or plant types to be evaluated;
- identification and / or selection of the microorganism(s) and/or composition(s);
- acquisition of the microorganism(s) and/or composition(s); and
- associating the microorganism(s) and/or composition(s) with the plant material.

Preferably, the method comprises evaluating (or arranging for said evaluation of) said plant(s) in the presence of said microorganism(s) and/or composition(s).

The step of evaluating preferably comprises performing one or more of the steps of a method described herein, in particular embodiments a method of the first aspect, seventh aspect or eighth aspect of the invention.

The various steps identified above may be performed by a single entity although it is preferred that at least two parties are involved, a first which makes a request and a second which actions the request. Note that various agents may act for one or both parties and that varying levels of automation may be used. For example, in response to a particular request the microorganism(s) may be selected by a processor querying a database based on known microorganism associations for that or similar plant(s) with little or no input required from an operator.

Furthermore, the evaluation may be performed by the requesting party and / or in the first region. Performing the evaluation in the first region better ensures that the evaluation is accurate and that no unforseen environmental factors that may impact on the plant(s) or the microorganism(s) are not considered.

Following the evaluation or during the course thereof, the method preferably further comprises one or more of:
- receiving or sending one or more microorganisms (or at least the identity thereof) and/or composition(s) to the first region, including in combination with plant material; and
- growing said plant(s) or other plants (preferably having similar properties) in the first region in the presence of said microorganism(s) and/or composition(s).

The method of the second aspect may be embodied by a first party:
- identifying a need for an improvement in a plant(s);
- sending the identity thereof and / or relevant plant material to a second party together with any relevant information, and
- receiving plant material and / or one or more microorganisms and / or the identities thereof and/or composition(s).

The step of receiving is preferably performed following or as a result of an assessment of plant/microorganism and/or plant/composition associations. Preferably, the assessment is made using a method as described herein, in particular embodiments a method of the first aspect, the seventh aspect or the eighth aspect.

The method of the second aspect may additionally or alternatively be embodied by a second party:
- receiving an identity of a plant(s) and / or relevant plant material from a first party together with any relevant information, and
- sending plant material and / or one or more microorganism(s) and / or the identities thereof and/or composition(s) to the first party.

The step of sending is preferably performed following or as a result of an assessment of plant / microorganism and/or plant/composition associations. Preferably, the assessment is made using a method described herein, in particular embodiments a method of the first aspect, the seventh aspect or the eighth aspect.

According to a third aspect, there is provided a system for implementing the method of the second aspect.

The system of the third aspect preferably includes one or more of:
- means for receiving or transmitting an identity of one or more plants or plant types to be evaluated;
- means for receiving or transmitting plant material from one or more plants or plant types to be evaluated;
- means for identifying and / or selecting microorganism(s) and/or composition(s);
- means for acquiring the microorganism(s) and/or composition(s);
- means for associating the microorganism(s) and/or composition(s) with the plant material;
- means for evaluating said plant(s) in the presence of said microorganism(s) and/or composition(s);
- means for receiving or sending one or more microorganisms (or at least the identity thereof) and/or composition(s) to the first region, including in combination with plant material; and
- means for growing said plant(s) or other plants (preferably having similar properties) in the first region in the presence of said microorganism(s) and/or composition(s).

Means known to those skilled in the art may be used to provide the functionality required in the system of the third aspect. For example, conventional communication means, including the internet, may be used to convey the identities of plants / microorganisms; conventional carrier means may be used to convey the plant material / microorganisms/ composition(s); conventional means and processes may be used to associate a microorganism and/or composition with plant material and conventional means for evaluating said plant(s) and / or the plant / microorganism and/or plant/composition associations may be used.

According to a preferred embodiment, the system of the invention is embodied by a facility configured to transmit request(s) for an improvement in a plant(s) and subsequently to receive plant material and / or one or more microorganisms and / or the identities thereof, preferably following or as a result of an assessment of plant / microorganism associations. Preferably, the assessment is made using a method described herein, in particular embodiments a method of the first aspect, the seventh aspect, or the eighth aspect.

The system of the second aspect may additionally or alternatively be embodied by a facility configured to receive an identity of a plant(s) and / or relevant plant material from together with any relevant information; and send plant material and / or one or more microorganisms and / or the identities thereof and/or composition(s), preferably following or as a result of an assessment of plant / microorganism or plant/composition associations. Preferably, the assessment is made using a method described herein, in particular embodiments a method of the first aspect, the seventh aspect or the eighth aspect.

Accordingly to a fourth broad aspect of the invention, there is provided a microorganism selected or isolated by a method as herein before described.

In a fifth broad aspect of the invention, there is provided a method for the production of a composition to support plant growth and/or health, the method comprising the steps of a method herein before described and the additional step of combining the one or more microorganisms selected by the method with one or more additional ingredients.

In a sixth broad aspect of the invention, there is provided a composition comprising one or more microorganism of the fourth broad aspect or as prepared by a method of the fifth broad aspect.

In a seventh broad aspect of the invention there is provided a method for the selection of a composition which is capable of imparting one or more beneficial property to a plant, the method comprising at least the steps of:
a) culturing one or more microorganism in one or moremedia;
b) separating the one or more microorganism from the one or more media after a period of time to provide one or more composition;
c) subjecting one or more plant (including seeds, seedlings, cuttings, and/or propagules thereof) to the one or more composition;
d) selecting one or more composition if it is observed to impart one or more beneficial property to the one or more plants.

In an eighth broad aspect of the invention there is provided a method for the selection of one or more microorganisms which are capable of producing a composition which is capable of imparting one or more beneficial property to a plant, the method comprising at least the steps of:
a) culturing one or more microorganism in one or more media;
b) separating the one or more microorganism from the one or more media after a period of time to provide one or more composition;
c) subjecting one or more plant (including seeds, seedlings, cuttings, and/or propagules thereof) to the one or more composition;
d) selecting the one or more microorganisms associated with one or more composition observed to impart one or more beneficial property to the one or more plants.

It should be appreciated that the methods of the first, seventh and eighth aspects may be combined in any combination, including the methods being run concurrently or sequentially in any number of iterations, with compositions and/or microorganisms selected or isolated from the methods being used individually or combined and used in iterative rounds of any one of the methods. By way of example, a method of the seventh aspect may be performed and a composition selected. The selection of a composition indicates that the one or more microorganism separated from the media in step b) is desirable for imparting beneficial properties to the one or more plant (as the one or more microorganism is capable of producing a selected composition). The one or more microorganism may then be used in another round of a method of the first aspect, seventh aspect or eighth aspect. Alternatively, the combination of methods could be run in reverse. This could be repeated any number of times in any order and combination.

In a ninth broad aspect of the invention there is provided a composition obtained as a result of the methods of the seventh or eighth broad aspects of the invention.

In a tenth broad aspect of the invention there is provided a combination of two or more microorganisms selected or isolated by a method as herein before described.

In another aspect, the invention provides the use of one or more composition and/or microorganism identified by a method of the invention for imparting one or more beneficial property to one or more plant.

In one embodiment the one or more microorganism is chosen from the group consisting of the microorganisms listed in tables 2, 3, 5, 6 and 7.

In one embodiment, the one or more microorganisms is chosen from the group consisting of the microorganisms listed in tables 2, 3, and 7 and the beneficial property is improved growth in nitrogen deficient or nitrogen limited growth media. In one particular embodiment the microorganism is Duganella sp. In another particular embodiment, a combination of Arthrobacta sp, Duganella sp, Acinetobacter sp, Panteoa sp, and Stenotrophomonas sp is used.

In one embodiment, the one or more microorganisms is chosen from the group consisting of the microorganisms listed in tables 5 and 6 and the beneficial property is improved growth in growth media in which phosphate is present substantially only in insoluble form.

The invention may also be said broadly to consist in the parts, elements and features referred to or indicated in the specification of the application, individually or collectively, in any or all combinations of two or more of said parts, elements or features, and where specific integers are mentioned herein which have known equivalents in the art to which the invention relates, such known equivalents are deemed to be incorporated herein as if individually set forth.

### FIGURES

These and other aspects of the present invention, which should be considered in all its novel aspects, will become apparent from the following description, which is given by way of example only, with reference to the accompanying figures, in which:
- **Figure 1:**: shows a system according to an embodiment of the invention;
- **Figure 2:**: shows the process flow of a method of an embodiment of the invention;
- **Figure 3:**: shows, on the left, a plate before incubation with 5 µL wells of liquid microbial culture, and on the right the plate shows zones of clearing around putative phosphate solubilising microbes after 24 hrs incubation at 28 °C (further details are provided in Example 11B).

### PREFERRED EMBODIMENT(S)

The following is a description of the preferred forms of the present invention given in general terms. The invention will be further elucidated from the Examples provided hereafter.

In one aspect the invention relates to a method for the selection of one or more microorganism(s) which are capable of imparting one or more beneficial property to a plant. Such beneficial properties include, for example: improved growth, health and/or survival characteristics, resistance to pests and/or diseases, tolerance to growth in different geographical locations and/or different environmental biological and/or physical conditions. By way of example, the invention may allow for the identification of microorganisms which allow a plant to grow in a variety of different temperatures (including extreme temperatures), pH, salt concentrations, mineral concentrations, in the presence of toxins, and/or to respond to a greater extent to the presence of organic and/or inorganic fertilisers.

As used herein, "improved" should be taken broadly to encompass improvement of a characteristic of a plant which may already exist in a plant or plants prior to application of the invention, or the presence of a characteristic which did not exist in a plant or plants prior to application of the invention. By way of example, "improved" growth should be taken to include growth of a plant where the plant was not previously known to grow under the relevant conditions.

Broadly, the method comprises at least the steps of a) growing one or more plant in a growth medium in the presence of one or more microorganisms; b) applying one or more selective pressure during step a); c) selecting one or more plant following step b); and, d) isolating one or more microorganisms from said one or more plant selected in step c). The one or more plants, growth medium and one or more microorganisms may be provided separately and combined in any appropriate order prior to step a). The invention also provides an iterative method in which steps a) to d) may be repeated one or more times, wherein the one or more microorganisms isolated in step d) are used in step a) of the next cycle of the method. Whilst the same selective pressure(s) may be applied in each iteration of the method, different selective pressures may be applied in each iteration. In addition, the method may comprise a further step (or steps) which are conducted prior to step a) and include subjecting the one or more plant to a growth medium in the presence of one or more microorganisms but without a selective pressure. After a desired period, one or more microorganisms may be isolated from said one or more plant and can be used in step a) of the process. It should also be appreciated, that successive rounds of this step may be conducted, with the microorganisms isolated being used in the subsequent round of the process, before embarking on steps a) to d) as described above. Further, the inventors envisage an iterative method in which steps a) to d) are repeated one or more times, but interspersed with the step of subjecting the plants to a growth medium and one or more microorganisms and isolating the microorganisms, without applying a selective pressure.

It should be appreciated that, particularly in the case of the iterative methods of the invention, the methods do not require the identification of the microorganisms in the population isolated from the plant(s) in step d) nor do they require a determination of the beneficial properties of individual microorganisms or combinations of microorganisms isolated from the plant(s). However, identification and a determination of the beneficial properties could be conducted if desired. For example, it may be preferred in some cases to isolate and identify the microbes in the final step of a method of the invention to determine their safety for commercial use and to satisfy regulatory requirements.

In one embodiment, where two or more microorganisms are isolated in step d), the method may further comprise the steps of separating the two or more microorganisms into individual isolates, selecting two or more individual isolates, and then combining the selected two or more isolates. In one embodiment, the combined isolates may then be used in step a) of successive rounds of the method. By way of example, from two, three, four, five, six, seven, eight, nine or ten individual isolates may be combined. The inventors envisage an iterative method in which steps a) to d) are repeated one or more times, utilising these additional steps of separating, selecting and combining with each repeat of the method, or interspersed or otherwise combined with a method in which individual isolates are not selected and combined.

It is expected that these combinations will detect previously unknown, plant growth promoting, synergistic interactions between previously isolated microbes. Using the iterative steps a) to d) will drive the starting population of two or more microorganisms toward only the microbes that interact with the plant to impart a desired change in the phenotype.

Selection of individual isolates may occur on the basis of any appropriate selection criteria. For example, it may be random, it may be based on the beneficial property or properties observed by performing a method of the invention or, where information about the identity of the microorganism is known, it may be on the basis that the microorganism has previously been recognised to have a particular beneficial property.

In addition, two or more methods of the invention may be performed separately or in parallel and the microorganisms that result from each method combined into a single composition. For example, two separate methods may be performed, one to identify microorganisms capable of imparting one or more first beneficial property, and a second to identify microorganisms capable of imparting one or more second beneficial property. The separate methods may be directed to identifying microorganisms having the same beneficial property or having distinct beneficial properties. The selective pressure applied in the separate methods may likewise be the same or different. Similarly, the microorganisms and plants used in the separate methods may be the same or different. If further optimisation of the microorganisms is desired, the single composition of microorganisms may be applied to one or more further rounds of a method of the invention. Alternatively, the single composition of microorganisms may be used, as desired, to confer the relevant properties to plant crops, without further optimisation. Combining two or more methods of the invention in this way allows for the selection and combination of microorganisms which may ordinarily be separated by time and/or space in a particular environment.

It should be further appreciated that where one or more microorganism(s) forms an association with a plant that allows vertical transmission from one generation or propagule to the next, step d) may be absent from the method or substituted by the step of multiplying the selected plant(s) from step c), as will be discussed further herein after.

Further methods and aspects of the invention are described herein after.

### Microorganisms

As used herein the term "microorganism" should be taken broadly. It includes but is not limited to the two prokaryotic domains, Bacteria and Archaea, as well as eukaryotic fungi and protists. By way of example, the microorganisms may include Proteobacteria (such as *Pseudomonas, Enterobacter, Stenotrophomonas, Burkholderia, Rhizobium, Herbaspirillum, Pantoea, Serratia, Rahnella, Azospirillum, Azorhizobium, Azotobacter, Duganella, Delftia, Bradyrhizobiun, Sinorhizobium* and *Halomonas*), Firmicutes (such as *Bacillus, Paenibacillus, Lactobacillus, Mycoplasma,* and *Acetobacterium*), Actinobacteria (such as *Streptomyces, Rhodococcus, Microbacterium,* and *Curtobacterium*), and the fungi Ascomycota (such as *Trichoderma, Ampelomyces, Coniothyrium, Paecoelomyces, Penicillium, Cladosporium, Hypocrea, Beauveria, Metarhizium, Verticullium, Cordyceps, Pichea*, and *Candida,* Basidiomycota (such as *Coprinus, Corticium,* and *Agaricus*) and Oomycota (such as *Pythium, Mucor,* and *Mortierella*)*.*

In a particularly preferred embodiment, the microorganism is an endophyte or an epiphyte or a microorganism inhabiting the plant rhizosphere.

Microorganisms of use in the methods of the present invention may be collected from any source.

In one embodiment, the microorganism are obtained from any general terrestrial environment, including its soils, plants, fungi, animals (including invertebrates) and other biota, including the sediments, water and biota of lakes and rivers; from the marine environment, its biota and sediments (for example sea water, marine muds, marine plants, marine invertebrates (for example sponges), marine vertebrates (for example, fish)); the terrestrial and marine geosphere (regolith and rock, for example crushed subterranean rocks, sand and clays); the cryosphere and its meltwater; the atmosphere (for example, filtered aerial dusts, cloud and rain droplets); urban, industrial and other man-made environments (for example, accumulated organic and mineral matter on concrete, roadside gutters, roof surfaces, road surfaces).

In another embodiment the microorganisms are collected from a source likely to favour the selection of appropriate microorganisms. By way of example, the source may be a particular environment in which it is desirable for other plants to grow. In another example, the source may be a plant having one or more desirable traits, for example a plant which naturally grows in a particular environment or under certain conditions of interest. By way of example, a certain plant may naturally grow in sandy soil or sand of high salinity, or under extreme temperatures, or with little water, or it may be resistant to certain pests or disease present in the environment, and it may be desirable for a commercial crop to be grown in such conditions, particularly if they are, for example, the only conditions available in a particular geographic location. By way of further example, the microorganisms may be collected from commercial crops grown in such environments, or more specifically from individual crop plants best displaying a trait of interest amongst a crop grown in any specific environment, for example the fastest-growing plants amongst a crop grown in saline-limiting soils, or the least damaged plants in crops exposed to severe insect damage or disease epidemic. The microorganisms may be collected from a plant of interest or any material occurring in the environment of interest, including fungi and other animal and plant biota, soil, water, sediments, and other elements of the environment as referred to previously.

In certain embodiments, the microorganisms are sourced from previously performed methods of the invention (for example, the microorganisms isolated in step d) of the method), including combinations of individual isolates separated from two or more microorganisms isolated in step d) or combinations of microorganisms resulting from two or more separately performed methods of the invention.

While the invention obviates the need for pre-existing knowledge about a microorganism's desirable properties with respect to a particular plant species, in one embodiment a microorganism or a combination of microorganisms of use in the methods of the invention may be selected from a pre-existing collection of individual microbial species or strains based on some knowledge of their likely or predicted benefit to a plant. For example, the microorganism may be predicted to: improve nitrogen fixation; release phosphate from the soil organic matter; release phosphate from the inorganic forms of phosphate (e.g. rock phosphate); "fix carbon" in the root microsphere; live in the rhizosphere of the plant thereby assisting the plant in absorbing nutrients from the surrounding soil and then providing these more readily to the plant; increase the number of nodules on the plant roots and thereby increase the number of symbiont nitrogen fixing bacteria (e.g. *Rhizobium* species) per plant and the amount of nitrogen fixed by the plant; elicit plant defensive responses such as ISR (induced systemic resistance) or SAR (systemic acquired resistance) which help the plant resist the invasion and spread of pathogenic microorganisms; compete with microorganisms deleterious to plant growth or health by antagonism, or competitive utilisation of resources such as nutrients or space.

In one embodiment a microorganism or combination of microorganisms is selected from a pre-existing collection of individual microbial species or strains that provides no knowledge of their likely or predicted benefit to a plant. For example, a collection of unidentified microorganisms isolated from plant tissues without any knowledge of their ability to improve plant growth or health, or a collection of microorganisms collected to explore their potential for producing compounds that could lead to the development of pharmaceutical drugs.

In one embodiment, the microorganisms are isolated from the source material (for example, soil, rock, water, air, dust, plant or other organism) in which they naturally reside. The microorganisms may be provided in any appropriate form, having regard to its intended use in the methods of the invention. However, by way of example only, the microorganisms may be provided as an aqueous suspension, gel, homogenate, granule, powder, slurry, live organism or dried material. The microorganisms may be isolated in substantially pure or mixed cultures. They may be concentrated, diluted or provided in the natural concentrations in which they are found in the source material. For example, microorganisms from saline sediments may be isolated for use in this invention by suspending the sediment in fresh water and allowing the sediment to fall to the bottom. The water containing the bulk of the microorganisms may be removed by decantation after a suitable period of settling and either applied directly to the plant growth medium, or concentrated by filtering or centrifugation, diluted to an appropriate concentration and applied to the plant growth medium with the bulk of the salt removed. By way of further example, microorganisms from mineralized or toxic sources may be similarly treated to recover the microbes for application to the plant growth material to minimise the potential for damage to the plant.

In another embodiment, the microorganisms are used in a crude form, in which they are not isolated from the source material in which they naturally reside. For example, the microorganisms are provided in combination with the source material in which they reside; for example, as soil, or the roots or foliage of a plant. In this embodiment, the source material may include one or more species of microorganisms.

It is preferred that a mixed population of microorganisms is used in the methods of the invention.

In embodiments of the invention where the microorganisms are isolated from a source material (for example, the material in which they naturally reside), any one of a number of standard techniques which will be readily known to skilled persons. However, by way of example, these in general employ processes by which a solid or liquid culture of a single microorganism can be obtained in a substantially pure form, usually by physical separation on the surface of a solid microbial growth medium or by volumetric dilutive isolation into a liquid microbial growth medium. These processes may include isolation from dry material, liquid suspension, slurries or homogenates in which the material is spread in a thin layer over an appropriate solid gel growth medium, or serial dilutions of the material made into a sterile medium and inoculated into liquid or solid culture media.

Whilst not essential, in one embodiment, the material containing the microorganisms may be pre-treated prior to the isolation process in order to either multiply all microorganisms in the material, or select portions of the microbial population, either by enriching the material with microbial nutrients (for example, nitrates, sugars, or vegetable, microbial or animal extracts), or by applying a means of ensuring the selective survival of only a portion of the microbial diversity within the material (for example, by pasteurising the sample at 60°C-80°C for 10 -20 minutes to select for microorganisms resistant to heat exposure (for example, bacilli), or by exposing the sample to low concentrations of an organic solvent or sterilant (for example, 25% ethanol for 10 minutes) to enhance the survival of actinomycetes and spore-forming or solvent-resistant microorganisms). Microorganisms can then be isolated from the enriched materials or materials treated for selective survival, as above.

In a preferred embodiment of the invention endophytic or epiphytic microorganisms are isolated from plant material. Any number of standard techniques known in the art may be used and the microorganisms may be isolated from any appropriate tissue in the plant, including for example root, stem and leaves, and plant reproductive tissues. By way of example, conventional methods for isolation from plants typically include the sterile excision of the plant material of interest (e.g. root or stem lengths, leaves), surface sterilisation with an appropriate solution (e.g. 2% sodium hypochlorite), after which the plant material is placed on nutrient medium for microbial growth (see, for example, Strobel G and Daisy B (2003) Bioprospecting for microbial endophytes and their natural products. Microbiology and Molecular Biology Reviews 67 (4): 491-502; Zinniel DK et al. (2002) Isolation and characterisation of endophytic colonising bacteria from agronomic crops and prairie plants. Applied and Environmental Microbiology 68 (5): 2198-2208). In one preferred embodiment of the invention, the microorganisms are isolated from root tissue. Further methodology for isolating microorganisms from plant material are detailed herein after.

As used herein, "isolate", "isolated" and like terms should be taken broadly. These terms are intended to mean that the one or more microorganism(s) has been separated at least partially from at least one of the materials with which it is associated in a particular environment (for example soil, water, plant tissue). "Isolate", "isolated" and like terms should not be taken to indicate the extent to which the microorganism(s) has been purified.

As used herein, "individual isolates" should be taken to mean a composition or culture comprising a predominance of a single genera, species or strain of microorganism, following separation from one or more other microorganisms. The phrase should not be taken to indicate the extent to which the microorganism has been isolated or purified. However, "individual isolates" preferably comprise substantially only one genera, species or strain of microorganism.

### Plants

Any number of a variety of different plants, mosses and lichens may be used in the methods of the invention. In preferred embodiments, the plants have economic, social and/or environmental value. For example, the plants may include those of use: as food crops; as fibre crops; as oil crops; in the forestry industry; in the pulp and paper industry; as a feedstock for biofuel production; and/or, as ornamental plants. The following is a list of non-limiting examples of the types of plants the methods of the invention may be applied to:

### Food crops:

- *Cereals* (maize, rice, wheat, barley, sorghum, millet, oats, rye, triticale, buckwheat);
- *leafy vegetables* (brassicaceous plants such as cabbages, broccoli, bok choy, rocket; salad greens such as spinach, cress, lettuce);
- *fruiting and flowering vegetables* (e.g. avocado, sweet corn, artichokes, curcubits e.g. squash, cucumbers, melons, courgettes , pumpkins; solononaceous vegetables/fruits e.g. tomatoes, eggplant, capsicums);
- *podded vegetables* (groundnuts, peanuts, peas, soybeans, beans, lentils, chickpea, okra);
- *bulbed and stem vegetables* (asparagus, celery, *Allium* crops e.g garlic, onions, leeks);
- *roots and tuberous vegetables* (carrots, beet, bamboo shoots, cassava, yams, ginger, Jerusalem artichoke, parsnips, radishes, potatoes, sweet potatoes, taro, turnip, wasabi);
- *sugar crops* including sugar beet (*Beta vulgaris*), sugar cane (*Saccharum officinarum*);
- *crops grown for the production of non-alcoholic beverages and stimulants* (coffee, black, herbal and green teas, cocoa, tobacco);
- *fruit crops* such as true berry fruits (e.g. kiwifruit, grape, currants, gooseberry, guava, feijoa, pomegranate), citrus fruits (e.g. oranges, lemons, limes, grapefruit), epigynous fruits (e.g. bananas, cranberries, blueberries), aggregate fruit (blackberry, raspberry, boysenberry), multiple fruits (e.g. pineapple, fig), stone fruit crops (e.g. apricot, peach, cherry, plum), pip-fruit (e.g. apples, pears) and others such as strawberries, sunflower seeds;
- *culinary and medicinal herbs* e.g. rosemary, basil, bay laurel, coriander, mint, dill, *Hypericum,* foxglove, alovera, rosehips);
- *crop plants producing spices* e.g. black pepper, cumin cinnamon, nutmeg, ginger, cloves, saffron, cardamom, mace, paprika, masalas, star anise;
- *crops grown for the production of nuts* e.g. almonds and walnuts, Brazil nut, cashew nuts, coconuts, chestnut, macadamia nut, pistachio nuts; peanuts, pecan nuts;
- *crops grown for production of beers, wines and other alcoholic beverages* e.g grapes, hops;
- *oilseed crops* e.g. soybean, peanuts, cotton, olives, sunflower, sesame, lupin species and brassicaeous crops (e.g. canola/oilseed rape); and,
- *edible fungi* e.g. white mushrooms, Shiitake and oyster mushrooms;

### Plants used in pastoral agriculture:

- *legumes: Trifolium* species, *Medicago* species, and *Lotus* species; White clover (*T.repens*); Red clover (*T.pratense*); Caucasian clover (*T. ambigum*); subterranean clover (*T.subterraneum*); Alfalfa/Lucerne (*Medicago sativum*); annual medics; barrel medic; black medic; Sainfoin (*Onobrychis viciifolia*); Birdsfoot trefoil (*Lotus corniculatus*); Greater Birdsfoot trefoil (*Lotus pedunculatus*);
- *seed legumes*/*pulses* including Peas (*Pisum sativum*), Common bean (*Phaseolus vulgaris*), Broad beans (*Vicia faba*), Mung bean (*Vigna radiata*), Cowpea (*Vigna unguiculata*), Chick pea (*Cicer arietum*), Lupins (*Lupinus* species);
- *Cereals* including Maize/corn (*Zea mays*), Sorghum (*Sroghum spp.*), Millet (*Panicum miliaceum, P. sumatrense*), Rice (*Oryza sativa indica, Oryza sativa japonica*), Wheat (*Triticum sativa*), Barley (*Hordeum vulgare*), Rye (*Secale cereale*)*,* Triticale (*Triticum* X *Secale*), Oats (*Avena fatua*);
- *Forage and Amenity grasses:* Temperate grasses such as *Lolium* species; *Festuca* species; *Agrostis* spp., Perennial ryegrass (*Lolium perenne*); hybrid ryegrass (*Lolium hybridum*); annual ryegrass (*Lolium multiflorum*), tall fescue (*Festuca arundinacea*); meadow fescue (*Festuca pratensis);* red fescue (*Festuca rubra*); *Festuca ovina*; Festuloliums (*Lolium* X *Festuca* crosses); Cocksfoot (*Dactylis glomerata*); Kentucky bluegrass *Poa pratensis; Poa palustris; Poa nemoralis; Poa trivialis*; *Poa compresa*; *Bromus* species; Phalaris (*Phleum* species); *Arrhenatherum elatius*; *Agropyron* species; *Avena strigosa*; *Setaria italic;*
- *Tropical grasses* such as: *Phalaris* species; *Brachiaria* species; *Eragrostis* species; *Panicum* species; Bahai grass (*Paspalum notatum*); *Brachypodium* species; and,
- Grasses used for biofuel production such as Switchgrass (*Panicum virgatum*) and *Miscanthus* species;

### Fibre crops:

- cotton, hemp, jute, coconut, sisal, flax (Linum spp.), New Zealand flax (Phormium spp.); plantation and natural forest species harvested for paper and engineered wood fibre products such as coniferous and broadleafed forest species;

### Tree and shrub species used in plantation forestry and bio-fuel crops:

- Pine (*Pinus* species); Fir (*Pseudotsuga* species); Spruce (*Picea* species); Cypress (*Cupressus* species); Wattle (*Acacia* species); Alder (*Alnus* species); Oak species (*Quercus* species); Redwood (*Sequoiadendron* species); willow (*Salix* species); birch (*Betula* species); Cedar (*Cedurus* species); Ash (*Fraxinus* species); Larch (*Larix* species); *Eucalyptus* species; Bamboo (Bambuseae species) and Poplars (*Populus* species).

### Plants grown for conversion to energy, biofuels or industrial products by extractive, biological, physical or biochemical treatment:

- Oil-producing plants such as oil palm, jatropha, soybean, cotton, linseed;
- Latex-producing plants such as the Para Rubber tree, *Hevea brasiliensis* and the Panama Rubber Tree *Castilla elastica*;
- plants used as direct or indirect feedstocks for the production of biofuels i.e. after chemical, physical (e.g. thermal or catalytic) or biochemical (e.g. enzymatic pre-treatment) or biological (e.g. microbial fermentation) transformation during the production of biofuels, industrial solvents or chemical products e.g. ethanol or butanol, propane diols, or other fuel or industrial material including sugar crops (e.g. beet, sugar cane), starch-producing crops (e.g. C3 and C4 cereal crops and tuberous crops), cellulosic crops such as forest trees (e.g. Pines, Eucalypts) and Graminaceous and Poaceous plants such as bamboo, switch grass, miscanthus;
- crops used in energy, biofuel or industrial chemical production via gasification and/or microbial or catalytic conversion of the gas to biofuels or other industrial raw materials such as solvents or plastics, with or without the production of biochar (e.g. biomass crops such as coniferous, eucalypt, tropical or broadleaf forest trees, graminaceous and poaceous crops such as bamboo, switch grass, miscanthus, sugar cane, or hemp or softwoods such as poplars, willows; and,
- biomass crops used in the production of biochar;

### Crops producing natural products useful for the pharmaceutical, agricultural nutraceutical and cosmeceutical industries:

- crops producing pharmaceutical precursors or compounds or nutraceutical and cosmeceutical compounds and materials for example, star anise (shikimic acid), Japanese knotweed (resveratrol), kiwifruit (soluble fibre, proteolytic enzymes);

### Floricultural, Ornamental and Amenity plants grown for their aesthetic or environmental properties:

- Flowers such as roses, tulips, chrysanthemums;
- Ornamental shrubs such as Buxus, Hebe, Rosa, Rhododendron, Hedera
- Amenity plants such as Platanus, Choisya, Escallonia, Euphorbia, Carex
- Mosses such as sphagnum moss

### Plants grown for bioremediation:

- Helianthus, Brassica, Salix, Populus, Eucalyptus

It should be appreciated that a plant may be provided in the form of a seed, seedling, cutting, propagule, or any other plant material or tissue capable of growing. In one embodiment the seed may surface-sterilised with a material such as sodium hypochlorite or mercuric chloride to remove surface-contaminating microorganisms. In one embodiment, the propagule is grown in axenic culture before being placed in the plant growth medium, for example as sterile plantlets in tissue culture.

### Growth Medium

The term "growth medium" as used herein, should be taken broadly to mean any medium which is suitable to support growth of a plant. By way of example, the media may be natural or artificial including, but not limited to, soil, potting mixes, bark, vermiculite, hydroponic solutions alone and applied to solid plant support systems, and tissue culture gels. It should be appreciated that the media may be used alone or in combination with one or more other media. It may also be used with or without the addition of exogenous nutrients and physical support systems for roots and foliage.

In one embodiment, the growth medium is a naturally occurring medium such as soil, sand, mud, clay, humus, regolith, rock, or water. In another embodiment, the growth medium is artificial. Such an artificial growth medium may be constructed to mimic the conditions of a naturally occurring medium, however, this is not necessary. Artificial growth media can be made from one or more of any number and combination of materials including sand, minerals, glass, rock, water, metals, salts, nutrients, water. In one embodiment, the growth medium is sterile. In another embodiment, the growth medium is not sterile.

The medium may be amended or enriched with additional compounds or components, for example, a component which may assist in the interaction and/or selection of specific groups of microorganisms with the plant and each other. For example, antibiotics (such as penicillin) or sterilants (for example, quaternary ammonium salts and oxidizing agents) could be present and/or the physical conditions (such as salinity, plant nutrients (for example organic and inorganic minerals (such as phosphorus, nitrogenous salts, ammonia, potassium and micronutrients such as cobalt and magnesium), pH, and/or temperature) could be amended.

In certain embodiments of the invention, the growth medium may be pre-treated to assist in the survival and/or selection of certain microorganisms. For example, the medium may be pre-treated by incubating in an enrichment media to encourage the multiplication of endogenous microbes that may be present therein. By way of further example, the medium may be pre-treated by incubating in a selective medium to encourage the multiplication of specific groups of microorganisms. A further example includes the growth medium being pre-treated to exclude a specific element of the microbial assemblage therein; for example pasteurization (to remove spore-forming bacteria and fungi) or treatment with organic solvents such as various alcohols to remove microorganisms sensitive to these materials but allow the survival of actinomycetes and spore-forming bacteria, for example.

### Growth Conditions

In accordance with the methods of the invention one or more plant is subjected to one or more microorganism and a growth medium. The plant is preferably grown or allowed to multiply in the presence of the one or more microorganism(s) and growth medium. The microorganism(s) may be present in the growth medium naturally without the addition of further microorganisms, for example in a natural soil. The growth medium, plant and microorganisms may be combined or exposed to one another in any appropriate order. In one embodiment, the plant, seed, seedling, cutting, propagule or the like is planted or sown into the growth medium which has been previously inoculated with the one or more microorganisms. Alternatively, the one or more microorganisms may be applied to the plant, seed, seedling, cutting, propagule or the like which is then planted or sown into the growth medium (which may or may not contain further microorganisms). In another embodiment, the plant, seed, seedling, cutting, propagule or the like is first planted or sown into the growth medium, allowed to grow, and at a later time the one or more microorganisms are applied to the plant, seed, seedling, cutting, propagule or the like and/or the growth medium itself is inoculated with the one or more microorganisms.

The microorganisms may be applied to the plant, seedling, cutting, propagule or the like and/or the growth medium using any appropriate techniques known in the art. However, by way of example, in one embodiment, the one or more microorganisms are applied to the plant, seedling, cutting, propagule or the like by spraying or dusting. In another embodiment, the microorganisms are applied directly to seeds (for example as a coating) prior to sowing. In a further embodiment, the microorganisms or spores from microorganisms are formulated into granules and are applied alongside seeds during sowing. In another embodiment, microorganisms may be inoculated into a plant by cutting the roots or stems and exposing the plant surface to the microorganisms by spraying, dipping or otherwise applying a liquid microbial suspension, or gel, or powder. In another embodiment the microorganism(s) may be injected directly into foliar or root tissue, or otherwise inoculated directly into or onto a foliar or root cut, or else into an excised embryo, or radicle or coleoptile. These inoculated plants may then be further exposed to a growth media containing further microorganisms, however, this is not necessary.

In one embodiment the microorganisms infiltrate parts of the plant such as the roots, stems, leaves and/or reproductive plant parts (become endophytic), and/or grow upon the surface of roots, stems, leaves and/or reproductive plant parts (become epiphytic) and/or grow in the plant rhizosphere. In one preferred embodiment microorganism(s) form a symbiotic relationship with the plant.

The growth conditions used may be varied depending on the species of plant, as will be appreciated by persons skilled in the art. However, by way of example, for clover, in a growth room one would typically grow plants in a soil containing approximately 1/3^{rd} organic matter in the form of peat, 1/3^{rd} compost, and 1/3^{rd} screened pumice, supplemented by fertilisers typically containing nitrates, phosphates, potassium and magnesium salts and micronutrients and at a pH of between 6 and 7. The plants may be grown at a temperature between 22-24°C in an 16:8 period of daylight:darkness, and watered automatically.

### Selective Pressure

At a desired time during the period within which the plant is subjected to one or more microorganism and a growth medium, a selective pressure is applied. The selective pressure may be any biotic or abiotic factor or element which may have an impact on the health, growth, and/or survival of a particular plant, including environmental conditions and elements which plants may be exposed to in their natural environment or a commercial situation.

Examples of biotic selective pressures include but are not limited to organisms that are detrimental to the plant, for example, fungi, bacteria, viruses, insects, mites, nematodes, animals.

Abiotic selective pressures include for example any chemical and physical factors in the environment; for example, water availability, soil mineral composition, salt, temperature, alterations in light spectrum (e.g. increased UV light), pH, organic and inorganic toxins (for example, exposure to or changes in the level of toxins), metals, organic nutrients, inorganic nutrients, air quality, atmospheric gas composition, air flow, rain fall, and hail.

For example, the plant/microorganisms may be exposed to a change in or extreme salt concentrations, temperature, pH, higher than normal levels of atmospheric gases such as CO₂, water levels (including drought conditions or flood conditions), low nitrogen levels, provision of phosphorus in a form only available to the plant after microbial degradation, exposure to or changes in the level of toxins in the environment, soils with nearly toxic levels of certain minerals such as aluminates, or high winds.

In one embodiment the selective pressure is applied directly to the plant, the microorganisms and/or the growth medium. In another embodiment the selective pressure is applied indirectly to the plant, the microorganisms and/or the growth medium, via the surrounding environment; for example, a gaseous toxin in the air or a flying insect.

The selective pressure may be applied at any time, preferably during the time the plant is subjected to the one or more microorganism and growth medium. In one embodiment, the selective pressure is applied during for substantially the whole time during which a plant is growing and/or multiplying. In another embodiment, the selective pressure is applied at a discrete time point during growth and/or multiplication. By way of example, the selective pressure may be applied at different growth phases of the one or more plants which simulate a potential stress on the plant that might occur in a natural or commercial setting. For example, the inventor has observed that some pests attack plants only at specific stages of the plant's life. In addition, the inventor has observed that different populations of potentially beneficial microorganisms can associate with plants at different points in the plant's life. Simulating a pest attack on the plant at the relevant time point, may allow for the identification and isolation of microorganisms which may protect the plant from attack at that particular life stage. It should also be appreciated that the selective pressure may be present in the growth medium or in the general environment at the time the plant, seed, seedling, cutting, propagule or the like is planted or sown.

In one embodiment, the microbial population is exposed (prior to the method or at any stage of the method) to a selective pressure to enhance the probability that the eventually-selected plants will have microbial assemblages likely to have desired properties. For example, exposure of the microorganisms to pasteurisation before their addition to a plant growth medium (preferably sterile) is likely to enhance the probability that the plants selected for a desired trait will be associated with spore-forming microbes that can more easily survive in adverse conditions, in commercial storage, or if applied to seed as a coating, in an adverse environment. Another example is provided herein after in Example 11B, in which microorganisms were subjected to a media which allowed for selection of phosphate solubilising microbes. Such a step of applying a selective pressure to the microbial population may be referred to herein as an "enrichment step".

The plants may be grown and subjected to the selective pressure for any appropriate length of time before they are selected and harvested. By way of example only, the plants and any microorganisms associated with them may be selected and harvested at any time during the growth period of a plant, in one embodiment, any time after germination of the plant. In a preferred embodiment, the plants are grown or allowed to multiply for a period which allows one to distinguish between plants having desirable phenotypic features and those that do not. By way of general example wheat may be selected for improvements in the speed of foliar growth say after one month, but equally may be selected for superior grain yield on maturity of the seed head. The length of time a plant is grown depends on the timing required to express the plant trait that is desired to be improved by the invention, or the time required to express a trait correlated with the desired trait. For example, in the case of winter wheat varieties, mainly sown in the Northern Hemisphere, it may be important to select plants that display early tillering after exposure of seed to a growth medium containing microorganisms under conditions of light and temperature similar to experienced by the winter wheat seed in the Northern Hemisphere, since early tillering is a trait related to winter survival, growth and eventual grain yield in the summer. Or, a tree species may be selected for improved growth and health at 4-6 months as these traits are related to the health and growth rate and size of trees of 10 years later, an impractical period product development using this invention.

It should be appreciated that the methods of the invention may involve applying two or more selective pressures simultaneously or successively in step b).

### Selection

Typically, following exposure to the selective pressure, one or more plant is selected based on one or more phenotypic traits. However, selecting plants based on genotypic information is also envisaged (for example, the pattern of plant gene expression in response to the microorganisms).

By way of example, plants may be selected based on growth rate, size (including but not limited to weight, height, leaf size, stem size, or the size of any part of the plant), general health, survival, and/or tolerance to adverse physical environments. Further non-limiting examples include selecting plants based on: speed of seed germination; quantity of biomass produced; increased root, and/or leaf/shoot growth that leads to an increased yield (herbage or grain or fibre or oil) or biomass production; effects on plant growth that results in an increased seed yield for a crop, which may be particularly relevant in cereal crops such as wheat, barley, oats, rye, maize, rice, sorghum, oilseed crops such as soybean, canola, cotton, sunflower, and seed legumes such as peas, beans; effects on plant growth that result in an increased oil yield, which may be particularly relevant in oil seed crops such as soybean, canola, cotton, jatropha and sunflower; effects on plant growth that result in an increased fibre yield (e.g. in cotton, flax and linseed) or for effects that result in an increased tuber yield in crops such as potatoes and sugar beet; effects on plant growth that result in an increased digestibility of the biomass which may be particularly relevant in forage crops such as forage legumes (alfalfa, clovers, medics), forage grasses (*Lolium* species; *Festuca* species; *Paspalum* species; *Brachiaria* species; *Eragrostis* species), forage crops grown for silage such as maize and forage cereals (wheat, barley, oats); effects on plant growth which result in an increased fruit yield which may be particularly relevant to pip fruit trees (such as apples, pears, etc), berry fruits (such as strawberries, raspberries, cranberries), stone fruit (such as nectarines, apricots), and citrus fruit, grapes, figs, nut trees; effects on plant growth that lead to an increased resistance or tolerance disease including fungal, viral or bacterial diseases or to pests such as insects, mites or nematodes in which damage is measured by decreased foliar symptoms such as the incidence of bacterial or fungal lesions, or area of damaged foliage or reduction in the numbers of nematode cysts or galls on plant roots, or improvements in plant yield in the presence of such plant pests and diseases; effects on plant growth that lead to increased metabolite yields, for example in plants grown for pharmaceutical, nutraceutical or cosmeceutical purposes which may be particularly relevant for plants such as star anise grown for the production of shikimic acid critical for the production of anti-influenza drug oseltamivir, or the production of Japanese knotweed for the extraction of resveratrol, or the production of soluble fibre and dietary enzyme products from kiwifruit, or for example increased yields of "condensed tannins" or other metabolites useful for inhibiting the production of greenhouse gases such as methane in grazing animals; effects on plant growth that lead to improved aesthetic appeal which may be particularly important in plants grown for their form, colour or taste, for example the colour intensity and form of ornamental flowers, the taste of fruit or vegetable, or the taste of wine from grapevines treated with microorganisms; and, effects on plant growth that lead to improved concentrations of toxic compounds taken up or detoxified by plants grown for the purposes of bioremediation.

In certain embodiments of the invention, selection for a combination of plant traits may be desired; for example, in the embodiments of the invention which involve repeating the basic method steps and applying different selective pressures with each iteration of the method. This can be achieved in a number of ways. In one embodiment, multiple rounds of iterative improvement for one trait, e.g. superior growth in nematode infested soils, are maintained until an acceptable level of nematode resistance is attained. Similar, but completely separate rounds of selection are undertaken to identify microorganisms that can confer at least different desirable traits, for example for improved growth resulting from improved microbial soil phosphate utilisation, or improved growth resulting from increased tolerance to sucking insect pests. Such separate rounds of selection may be performed using an iterative or stacking approach or a combination of separate methods could be used, with the microorganisms that result from those separate rounds or methods being combined into a single composition. At this point the microorganism(s) could be developed into a product containing combinations of separately-fermented microorganisms each shown to improve a different plant attribute. In a further embodiment, the separately selected sets of microorganisms may be combined in sets of two or more and used in further methods of the invention. In another embodiment, the separately selected sets of microorganisms may be separated into individual isolates and then individual isolates combined in sets of two or more and used in further methods of the invention. In one embodiment, the combined microorganisms are applied to the plant and/or growth medium for the application of two or more selection pressures in the same iterative cycle. For example, in one combination, microorganisms able to improve plant growth in a medium containing low-levels of plant-available phosphorus are combined with microorganisms able to enhance plant growth in soils infested with plant parasitic nematodes. The combined microorganisms are then added to a plant growth medium with low levels of available phosphorus in which the plants are grown for a suitable period, nematodes applied and the plants are further grown until nematode damage can be expressed. The degree of nematode root damage and plant biomass is assessed non-destructively and microbes are isolated from the best-performing plants for use in a succeeding iteration. Similar iterative rounds may be continued until an acceptable level of plant growth is attained under both selective pressures. This approach will aid the selection of microbes that synergistically improve plant performance i.e. improve plant growth and nematode resistance to a degree better than that achieved if the microorganisms are applied simply as a combination of two separately-selected sets.

### Harvesting

Following selection, one or more plants are harvested and plant tissues may be examined to detect microorganisms forming associations with the plants (for example, endophytic, epiphytic or rhizospheric associations).

The one or more microorganisms may be isolated from any appropriate tissue of the plants selected; for example, whole plant, foliar tissue, stem tissue, root tissue, and/or seeds. In a preferred embodiment, the microorganisms are isolated from the root tissue, stem or foliar tissues and/or seeds of the one or more plants selected.

The microorganisms may be isolated from the plants using any appropriate methods known in the art. However, by way of example, methods for isolating endophytic microbes may include the sterile excision of the plant material of interest (e.g. root, stem lengths, seed), surface sterilisation with an appropriate solution (e.g. 2% sodium hypochlorite), after which the plant material is placed on nutrient medium for microbial outgrowth, especially filamentous fungi. Alternatively, the surface-sterilised plant material can be crushed in a sterile liquid (usually water) and the liquid suspension, including small pieces of the crushed plant material spread over the surface of a suitable solid agar medium, or media, which may or may not be selective (e.g. contain only phytic acid as a source of phosphorus). This approach is especially useful for bacteria and yeasts which form isolated colonies and can be picked off individually to separate plates of nutrient medium, and further purified to a single species by well-known methods. Alternatively, the plant root or foliage samples may not be surface sterilised but only washed gently thus including surface-dwelling epiphytic microorganisms in the isolation process, or the epiphytic microbes can be isolated separately, by imprinting and lifting off pieces of plant roots, stem of leaves on to the surface of an agar medium and then isolating individual colonies as above. This approach is especially useful for bacteria and yeasts, for example. Alternatively, the roots may be processed without washing off small quantities of soil attached to the roots, thus including microbes that colonise the plant rhizosphere. Otherwise, soil adhering to the roots can be removed, diluted and spread out onto agar of suitable selective and non-selective media to isolate individual colonies of rhizospheric microbes. Further exemplary methodology can be found in: Strobel G and Daisy B (2003) Bioprospecting for microbial endophytes and their natural products. Microbiology and Molecular Biology Reviews 67 (4): 491-502; Zinniel DK et al. (2002) Isolation and characterisation of endophytic colonising bacteria from agronomic crops and prairie plants. Applied and Environmental Microbiology 68 (5): 2198-2208), Manual of Environmental Microbiology, Hurst et al., ASM Press, Washington DC.

In embodiments of the invention where two or more microorganism are isolated from plant material and then separated into individual isolates, any appropriate methodology for separating one or more microorganism from each other may be used. However, by way of example, microbial extracts prepared from plant material could be spread on agar plates, grown at an appropriate temperature for a suitable period of time and the resulting microbial colonies subsequently selected and grown in an appropriate media (for example, streaked onto fresh plates or grown in a liquid medium). The colonies may be selected based on morphology or any other appropriate selection criteria as will be understood in the art. By way of further example, selective media could be used. Further methods are described in the Examples section herein after.

The one or more microorganisms may be harvested from the plants at any appropriate time point. In one embodiment they are harvested at any time after germination of the plant. For example, they can be isolated from the period shortly after germination (where survival in the first few days after germination is an issue, for example with bacterial and fungal root and collar rots), then at any stage after that, depending on the timing required for a plant to grow in order to evidence a discriminatory benefit that enables it's selection from the plant population (for example, to discriminate say the top 10 of 200 plants) exposed to the selective pressure.

The inventor has observed that different microorganisms may associate with a plant at different stages of the plant's life. Accordingly, harvesting a plant at different time points may result in selection of a different population of microorganisms. Such microorganisms may be of particular benefit in improving plant condition, survival and growth at critical times during its life: by way of example, as mentioned herein before, a plant may be susceptible to attack by nematodes at discrete time points during its life and the invention may be used to identify and isolate a population of microorganisms which may increase resistance to such attack at that particular life stage.

In another embodiment of the invention, in the case of microorganisms that form an association with a plant that allows vertical transmission from one generation or propagule to the next (for example seed-endophytic or -epiphytic associations, or endophytic and epiphytic associations with plants/propagules multiplied vegetatively) the microorganisms may not be isolated from the plant(s). The target plant itself may be multiplied by seed or vegetatively (along with the associated microorganisms) to confer the benefit(s) to "daughter" plants of the next generation or multiplicative phase.

### Stacking

The inventor envisages advantages being obtained by stacking selective pressures in repeated rounds of the method of the invention. This may allow for the isolation of a population of microorganisms that may assist a plant in surviving in a number of different environmental conditions, resisting a number of different diseases and attack by a number of different organisms, for example.

In this embodiment of the invention the one or more microorganisms isolated from the one or more plants selected following exposure to the selective pressure, as previously described, is used in a second round or cycle of the method; ie the microorganisms isolated from the selected plants are provided, along with one or more plants and a growth medium, a selective pressure is applied, plants are selected at a desired time and microorganisms are isolated from the selected plants. The microorganisms isolated from the second round of the method may then be used in a subsequent round, and so on and so on.

In one embodiment, the selective pressure applied in each repeat of the method is different. For example, in the first round the pressure may be a particular soil pH and in the second round the pressure may be nematode attack. However, in other embodiments of the invention, the selective pressure applied in each round may be the same. It could also be the same but applied at differing intensities with each round. For example, in the first round the selective pressure may be a particular concentration of salt present in the soil. In the second round, the selective pressure may be a higher concentration of salt present in the soil. In one embodiment, the selective pressure is increased in successive rounds in a pattern that may be linear, stepped or curvilinear. For example in round 1 of an iterative selective process wheat plus microorganisms may be exposed to 100 mM NaCl, in the second to 110 mM salt, in the third to 120 mM salt, thus increasing the selective pressure on the plants as adaptation occurs via improved plant/microorganism associations. Alternatively, it may be advantageous to maintain a selective pressure of 120mM for several rounds to allow for a slower adjustment in the microbial population balance underlying improvements in the ability of wheat to grow productively in a higher salt environment.

In one embodiment, the selective pressure may be separated disjunctively from a specific step of the iterative process, particularly the first round of an iterative cycle. For example in round one the selective pressure may not be applied at all. But after the microorganisms have been isolated from the selected plants after exposure for a relevant period to a growth medium and microorganisms in round 1, they are applied to the plant growth medium along with the plant, seed, seedling, cutting, propagule or the like for round 2. After an appropriate time a selective pressure is applied in round 2 and in successive rounds. This type of selection may be especially relevant for selection factors that severely diminish the plant tissue that is the target of the selection. For example nematodes are especially destructive of root tissue and it may be advantageous to allow particular microbes to multiply to high levels on, in, or around the roots in round 1 to allow high concentrations of microorganisms from the roots of plants selected in round 1 to be applied to the growth medium in round 2.

It should be appreciated that each successive round of the iterative method of the invention may be interspersed with a round in which no selective pressure is applied, as previously mentioned herein before.

It should also be appreciated that in certain embodiments of the invention, where one or more microorgansim(s) forms an endophytic or epiphytic relationship with a plant that allows vertical transmission from one generation or propagule to the next the microorganisms need not be isolated from the plant(s). The target plant itself may be multipled by seed or vegetatively (along with the associated microorganisms) to confer the benefit(s) to "daughter" plants of the next generation or multiplicative phase.

It should further be appreciated that two or more selective pressures may be applied with each iteration of the method.

### Isolated microorganisms and compositions containing same

In addition to the methods described herein before, the invention relates to microorganisms isolated by such methods and compositions comprising such microorganisms. In its simplest form, a composition comprising one or more microorganisms includes a culture of living microorganism, and microorganisms in a live but inactive state(s), including frozen, lyophilised or dried cultures. However, the compositions may comprise other ingredients, as discussed below.

The invention should also be understood to comprise methods for the production of a composition to support plant growth and/or health, the method comprising the steps of a method herein before described and the additional step of combining the one or more microorganisms with one or more additional ingredients.

A "composition to support plant growth and/or health" should be taken broadly to include compositions which may assist the growth, general health and/or survival of a plant. The phrase should not be taken to imply that the composition is able to support plant growth and/or health on its own. However, in one embodiment the compositions are suitable for this purpose. Exemplary compositions of this aspect of the invention include but are not limited to plant growth media, plant mineral supplements and micronutrients, composts, fertilisers, potting mixes, insecticides, fungicides, media to protect against infection or infestation of pests and diseases.

Skilled persons will readily appreciate the types of additional ingredients that may be combined with the one or more microorganisms, having regard to the nature of the composition that is to be made, the microorganisms to be used, and/or the method of delivery of the composition to a plant or its environment. However, by way of example, the ingredients may include liquid and/or solid carriers, microbial preservatives, additives to prolong microbial life (such as gels and clays), wettable powders, granulated carriers, soil, sand, agents known to be of benefit to microbial survival and the growth and general health of a plant, peat, organic matter, organic and inorganic fillers, other microorganisms, wetting agents, organic and inorganic nutrients, and minerals.

Such compositions can be made using standard methodology having regard to the nature of the ingredients to be used.

Compositions developed from the methods of the invention may be applied to a plant by any number of methods known to those skilled in the art. These include for example: sprays; dusts; granules; seed-coating; seed spraying or dusting upon application; germinating the seed in a bed containing suitable concentrations of the composition prior to germination and planting out of the seedlings; prills or granules applied next to the seed or plant during sowing or planting, or applied to an existing crop through a process such as direct drilling; application to plant cuttings or other vegetative propagules by dipping the cut surface or the propagule into liquid or powdered microbial substrate prior to planting; application to the soil as a "soil treatment" in the form of a spray, dust, granules or composted composition that may or may not be applied with plant fertilisers prior to or after sowing or planting of the crop; application to a hydroponic growth medium; inoculation into plant tissues under axenic conditions via injection of compositions or otherwise inoculated via a cut in such tissues, for the subsequent establishment of an endophytic relationship with the plant that extends to the seed, or propagative tissues, such that the plant can be multiplied via conventional agronomic practice, along with the endophytic microbe providing a benefit(s) to the plant.

### Method of producing alternative compositions

When microorganisms are cultured they may produce one or more metabolites and which are passed into the media in which they reside. Such metabolites may confer beneficial properties to plants.

Accordingly, the invention also provides a method of producing a composition capable of imparting one or more beneficial property to a plant, for example to support plant growth and/or health, or to identify microorganisms that are capable of producing such a composition. In one embodiment, the composition is substantially free of microorganisms.

In one embodiment the method for the selection of a composition capable of imparting one or more beneficial property to a plant, comprises at least the steps of:
a) culturing one or more microorganism in one or more media;
b) separating the one or more microorganism from the one or more media after a period of time to provide one or more composition;
c) subjecting one or more plant (including seeds, seedlings, cuttings, and/or propagules thereof) to the one or more composition;
d) selecting one or more composition if it is observed to impart one or more beneficial property to the one or more plants.

In one embodiment the method for the selection of one or more microorganism which is capable of producing a composition which is capable of imparting one or more beneficial property to a plant, comprises at least the steps of:
a) culturing one or more microorganism in one or more media;
b) separating the one or more microorganism from the one or more media after a period of time to provide one or more composition;
c) subjecting one or more plant (including seeds, seedlings, cuttings, and/or propagules thereof) to the one or more composition;
d) selecting the one or more microorganisms associated with one or more composition observed to impart one or more beneficial property to the one or more plants.

In this method, microorganisms from any source (as described herein before, for example) are cultured in two or more (preferably a large number, for example, from at least approximately 10 to up to approximately 1000) mixed cultures using media that can support the growth of a wide variety of microorganisms. Any appropriate media known in the art may be used. However, by way of example, fermentation media, TSB (tryptic soy broth), Luria-Bertani (LB) broth, or Reasoner's (R2A) broth. In another embodiment, selective or enrichment media which are able to support the growth of microorganisms with an array of separate but desirable properties may be used. By way of example, the enrichment media referred to elsewhere herein may be used.

The microorganisms may be cultured in the media for any desired period. Following culture, the microorganisms are separated from the media and stored for later use. A separate composition also results. One or more plants in a suitable growth medium are then subjected to the composition (using any known methodology, or methodology as described herein before). After a period of time, growth of plants is assessed and plants selected (as described herein before, for example). Plants are preferably selected on the basis of size. However, other selection criteria as referred to herein may be used.

In one embodiment, the microorganism(s) producing the subset of compositions associated with the selected plants are recovered from storage. Two or more separate cultures of the microorganisms may then be mixed together and separated into two or more sub-cultures grown in two or more different media.

This process can be repeated iteratively as many times as is deemed efficacious, with progressive steps refining down to fewer media and a narrower diversity of microorganisms until a desirable effect on the growth plants is achieved with a mixture of microbes that can be identified, grown and stored indefinitely as a standard starting inoculum for the production the composition.

An example of an embodiment of this aspect of the invention is provided in Example 7 herein after.

Compositions of this aspect of the invention may be used or formulated on their own or combined with one or more additional ingredients.

It should be appreciated that the general methodology described herein before may be applicable to this aspect of the invention, including but not limited to growth media, plants, microorganisms, selective pressures, timing, iterative processing, and combinations thereof.

### Additional Methodology

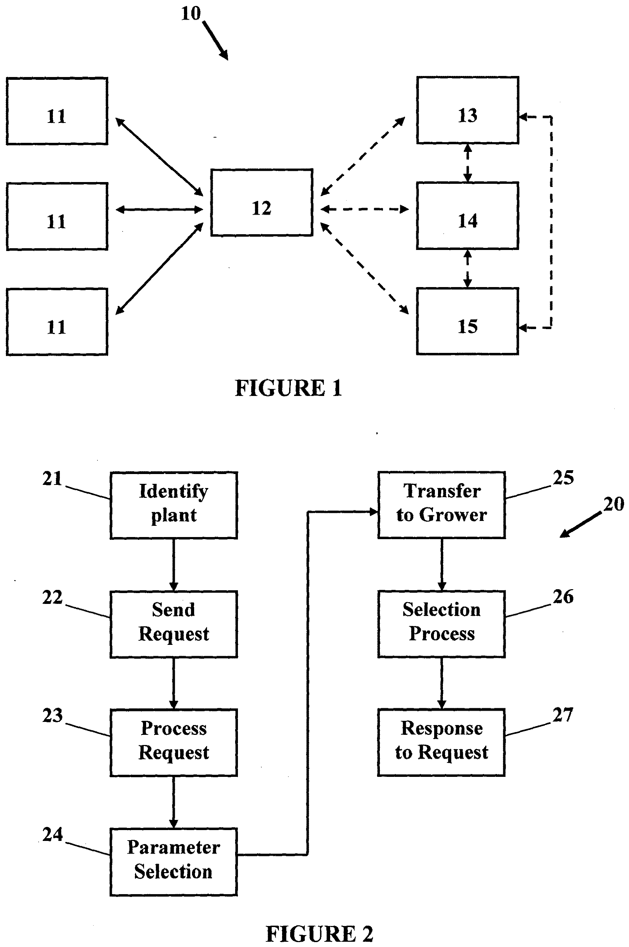

Figure 1 shows a system 10 according to an embodiment of the invention. System 10 includes requestors 11, request processor 12, growing facility 13, database or library 14 and depository 15.

Figure 2 provides a flow chart illustrating a method 20 according to an embodiment of the invention. The steps shown in Figure 2 will be described with reference to the system 10 shown in Figure 1.

This aspect of the invention is described in terms of identifying one or more microorganism that may impart one or more desired properties to one or more plants, and in some cases with particular reference to the first aspect of the invention. However, it should be appreciated that it is equally applicable to the identification of one or more compositions that may impart one or more desired property to one or more plant, or one or more microorganism that produces a composition that may impart one or more desired property to one or more plant, as herein before described, and summarised in the seventh and eighth aspects of the invention. Accordingly, unless the context requires otherwise, reference to the first aspect of the invention should be taken to also include reference to the seventh and eighth aspects of the invention, and reference to one or more microorganism should be taken to include reference to one or more composition.

The method begins at step 21 with a requestor 11 identifying a plant (or a class or group of plants). Reasons why particular plants or types of plants may be identified will be apparent to those skilled in the art. However, by way of example, it may have been found that a plant noted in general for having a high growth rate is growing at lower rates or not at all, there may simply be a desire to improve on existing growth rates or there may be a desire to introduce a plant to a different climate / environment / geographical region. The invention is not limited to conferring improvements to particular plant(s) and may be used to inhibit growth or otherwise adversely affect the plant(s).

At step 22, the requestor 11 sends the plant and / or the identity thereof to a request processor 12. The requestor 11 may provide further relevant information such as why or what properties they are seeking to improve. While only one request processor 12 is shown, it will be appreciated that more than one may be provided in the system 10.

Where a requestor 11 identifies a class or group of plants, more than one plant variety may be evaluated. Alternatively or additionally, selection of a one or more plant variety may be made elsewhere within the system 10 based on the group or class identified, including following evaluation of different varieties including using different microorganisms in accordance with methods of the invention.

Requests may conveniently be received over the internet via a web browser, although the invention is not limited thereto. Use of a web browser may additionally or alternatively be used to enable a requestor 11 to view reports on the progress being made in response to their request. For example, measures of growth may be provided.

At step 23, the request processor 12 receives and processes the request, essentially by initiating the performance of the method for the selection of one or more microorganism according to the first aspect of the invention. Note that the request processor 12 may or may not actively perform the method of the first aspect, or may only perform parts thereof. According to particular embodiments, the request processor 12 may act as an intermediary or agent between the requestor 11 and the parties able to perform the method of the first aspect. Also, different arrangements may be made in response to different requests. For example, for one request, the environment around the request processor 12 may be suitable for evaluating a particular plant but unsuitable for another, requiring the assistance of a third party facility. This could be due to a desire to test in a particular soil type, altitude or climate. Other factors will also be apparent although it is appreciated that "artificial" environments may be used. Furthermore, varying degrees of user interaction may take place at the request processor 12. According to one embodiment, a computer processor selects parameters or conditions for a study based on data input by a requestor 11. As will be appreciated, providing a structured information request may help to effect this, and where necessary, reference may be made to databases including database 14.

At step 24, parameters of the evaluation process are selected. For example, reference may be made to database 14 for microorganisms that may provide the desired improvement in the plant(s). While little data to date has been provided in the art on microorganisms having beneficial associations with particular plant varieties, this will be improved upon through ongoing operation of the methods of the invention and stored in database 14. Other parameters such as plant type(s) and environmental conditions may also be selected.

At step 25, the request (or portions thereof) and evaluation parameters are sent to growing facility 13 which may obtain suitable microorganisms from depository 15. These may or may not have been previously identified. While only one growing facility 13 and one depository 15 are shown, it will be appreciated that the invention is not so limited. Furthermore, any two or more of request processor 12, growing facility 13, database 14 and depository 15 may be co-located and/or under the same control.

At step 26, a selection process is performed, preferably according to the selection method of the first aspect.

At step 27, a response is sent to the request. A response may be sent to the requestor 11 and / or to a third party and preferably includes at least one of at least a subset of the results generated at step 26, identification of plant(s), plant(s), identification of microorganism(s), microorganism(s), or plant(s) provided in association with microorganisms, namely those that have been shown to provide benefits at step 26.

At step 28, database 14 may be updated with results of the selection process of step 26. This step may be performed prior to step 27, including periodically or at other various stages which the selection process is conducted. Preferably, at least details of new beneficial associations between plant(s) and microorganisms are recorded. It will be appreciated that incompatible or less beneficial associations will also preferably be recorded, thereby over time building a knowledge framework of plants and microorganisms.

It will be appreciated that one or more of the steps of Figure 2 may be omitted or repeated. For example, growing facility 13 may generate results at step 26 and in response thereto, one or more of steps 21 to 26 may be repeated.

Thus, the invention provides means and methods to improve plant(s) (or growth or other characteristics thereof). This is achieved by enabling a requestor 11 in a first geographical region (e.g. country) or otherwise defined environment (e.g. by parameters or characteristics affecting growing conditions such as such soil salinity or acidity) to access microbiological biodiversity not present or of limited presence in the first region for the purposes of plant improvement in the first or another region. The other region may be or in a foreign country but may be otherwise defined by characteristics of that environment that affect a plant rather than being defined by political boundaries. Consequently, the invention may enable a requestor to obtain the beneficial effects of a particular microorganism(s) on a particular plant(s) in a first region, even though such microorganism(s) may not be present or are of limited presence in the first region.

An example implementation of the invention is provided below.
1. A company in say New Zealand (home company), enters into a contractual relationship with a second, say overseas, company (overseas company).
2. The overseas company agrees to send seeds, cuttings or other plant propagules (foreign cultivar) to the home company from plant cultivars adapted to the environment(s) in its own, or other foreign countries, in order to gain access to elements of New Zealand's terrestrial and marine microbial biodiversity that are able to form beneficial plant-microorganism associations with the foreign cultivar.
3. The nature of the benefit may encompass increased plant productivity, for example through any one or more of but not limited to: increased root or foliar mass, or through an increase in efficiency in nutrient utilisation through nitrogen fixation by diazatrophs such as Klebsiella or Rhizobium, or through release of plant nutrients from the soil, such as phosphates released soil through the production of microbial phytases, or through improved resistance to attack from pests and diseases spanning a broad range of nematodes, insects, microbial and virus diseases, or through improvements in the ability of the plant to resist adverse environmental conditions such as drought, salinity, extreme temperatures, toxic soil minerals, or through improvements in plant phenotype for example date of flowering, or changes in physical form e.g. colour frequency of root or foliar branching.
4. In New Zealand, the home company identifies which indigenous microorganisms can form an association with the foreign plant by exposing the seed to the microorganisms, with or without knowledge of their likely effects on the plant, by the method of germinating the seed and growing the plant in a growing material that ensures contact of the plant during its growth with indigenous microorganisms via seed coating, direct inoculation into the seed or germinating seedling and/or contamination of the growing medium. The invention is not limited to this arrangement or methodology. For example, it may be apparent that microorganisms present in soil other than in New Zealand may provide benefits and testing may be conducted in such regions in addition to or instead of New Zealand. Also, artificial environments may be created. Referring to the immediately prior example, this may be achieved by obtaining soil and / or microorganisms from such regions and conducting the tests in say New Zealand. As will be apparent, such embodiments may include provision for artificial control of climatic conditions among other parameters. Thus, the invention is not limited to conducting testing in a region based on its indigenous microorganisms - the microorganisms may be artificially introduced so as to conduct the testing elsewhere than in the microorganisms' natural environment.
5. The period of growth and the physical conditions under which they take place may vary widely according to plant species and specific plant improvement traits, including based on parameters desired or specified by the overseas company.
6. After the relevant period of plant growth the nature of possible plant-microorganisms associations is determined by microbiological assessment to determine whether microorganisms have formed an endophytic, epiphytic or rhizospheric association with the foreign crop. One or more of the previous steps may be repeated as required until a desired relationship is found.
7. Where such association(s) are demonstrated the microorganisms form a collection of (say New Zealand indigenous) microorganisms able to associate with the (say foreign) crop or plant.
8. In one embodiment of the invention, microbial isolates of the collection may, for example, be coated on to seeds, inoculated into seeds or seedlings, or inoculated into a growing medium that may or may not be sterile.
9. After a suitable period the plants are assessed for improved root and foliar growth and / or exposed to environmental stressors designed to identify the plant-microorganism associations most able to provide benefit to the plant in the manner desired by the overseas company.
10. Examples of such stressors or selection criteria are provided in 3 above, and where identical pests, diseases or other parameters of the second, overseas environment are not present in the home or test region (i.e., New Zealand in the example), similar microbial diseases, nematode and insect pests or other parameters most similar to those in the overseas environment and that may be considered acceptable to the overseas company may be selected. As mentioned in 4 above, the invention also includes introducing foreign material or creating otherwise artificial conditions in the home or test region.
11. Elite microorganisms providing commercially-significant benefit to the growth of the foreign cultivar are identified by this process and may be shipped to the overseas company for further testing and selection in the foreign environment.
12. In a further embodiment the overseas company will agree that microorganisms found on, or in, the seed, cuttings or propagules of the foreign cultivar will be added to the collection of the home company to enlarge the collection for use both on that cultivar or on other foreign cultivars received for similar testing from other (overseas) companies.

In an alternative embodiment, the microbial isolates able to form plant-microorganism associations with the foreign cultivar i.e., the collection, are sent to the second company for testing and selection, such that items 8-11 above are performed by and / or in the grounds of the second company. This may be performed by or under the control of the first company.

As a further alternative, rather than identifying and using predetermined microorganism(s) of a collection, the home company may simply expose the seed to indigenous microorganisms, with or without knowledge of their likely effects on the plant, for example by germinating the seed and growing the plant in a growing material that ensures contact of the plant during its growth with indigenous microorganisms via seed coating, direct inoculation into the seed or germinating seedling and / or contamination of the growing medium or otherwise. As will be apparent, the home company may additionally or alternatively arrange for similar testing in other regions, where the same or different microorganisms may be present. The period of growth and the physical conditions under which they take place may vary widely according to plant species and specific plant traits desired by the overseas company. After a period of plant growth the nature of possible plant-microorganism associations may be determined in a similar manner to that described above.

### EXAMPLES

The invention is now further described by the following non-limiting examples.

It should be appreciated that Examples 1, 2 and 3 may exemplify applications of the invention in greater detail than subsequent examples. However, it should be appreciated that similar methodology to that described in Examples 1, 2 and 3 may be used in the further examples.

### Example 1:

To identify microorganisms able to improve the growth of legumes, such as clover in the presence of plant parasitic nematodes:
*Step* 1. untreated clover seeds are planted in a wide variety of soils in small pots. After a suitable period of growth, say 2 months, the plant are washed out of the soil, and the microorganisms isolated from roots and stems/foliage, either as individual isolates in pure culture, or as mixed populations e.g. as a microbial suspension from an aqueous root crush and/or a stem/foliar crush.
*Step 2.* The microorganisms are then added to a plant growth medium into which untreated clover seeds are planted. Alternatively, the microorganism(s) are mixed into a suitable seed coating material e.g. a gel, and coated onto seeds before being planted into a similar plant medium. Alternatively, the seeds are geminated and then exposed to the microorganisms for a short period (usually between 1 - 24 hours to maximise the chance that the microbes may form an endophytic or epiphytic association with the germinating plant) and then planted into a similar growth medium. In each of these cases the growing medium may be initially sterile, although this is not essential and further microorganisms applied to the growth medium and/or plant. After a suitable period of clover growth (e.g. one month), between 1-1000 viable root-knot or cyst nematode eggs per gram of soil are added to the medium. After a period of further growth, e.g. 6 - 12 weeks, the plants are removed from the containers and gently washed clean, relative growth is assessed non-destructively (e.g. by image analysis of roots, stems/foliage), and assessments made of nematode damage.
*Step 3:* The plants least-affected by nematode exposure are selected, and their root and foliar microorganisms isolated and prepared as in Step 2. The process from step 2 to step 3 may then be repeated iteratively, with or without increasing numbers of nematodes applied to each pot to increase the selective pressure.
*Step 4:* after this iterative process has been conducted to the point at which improvement in the ability of the clover plants to grow in the in the presence of plant-parasitic nematodes is deemed to be sufficient, the best-performing plants are selected and the microorganisms associated with them are isolated and used to develop a commercial product that improves the growth of clover in nematode-infested soils.
*Step 5:* Typically, the development of such a product would entail the isolation of microorganisms to pure culture from the roots, stems and rhizosphere of plants selected in the final iterative step. Preferably, the identities and relative concentrations of microorganisms in root and foliar tissues would be determined as would the nature of their association with the clover plant (ie. endophytic and/or epiphytic and/or rhizospheric). Back-tests of single and combined microorganisms applied to clover and exposed to nematodes as above could be conducted to determine the relative contribution of each microbe to the observed plant benefit and to help optimise the final product.

The products developed from the method may comprise a single microorganism or a mixture of two or more microorganisms. Methods of product application to clover may include but not be limited to, seed-coating or dusting, application to seed at the time of planting via sprayed suspension, co-application as a granulated, powdered or composted microbial product. Alternatively, the microorganism(s) may be applied at any time after planting by way of a sprayed, granulated, powdered or composted microbial product. Alternatively, the product may be sprayed on to clover seed crops at a suitable time prior to, during or following flowering thereby infecting the seed or otherwise associating with it, enabling the seed to be sold as a seed-line that imbues the resultant clover plants with anti-nematode properties. Alternatively, microorganism(s) in the product may naturally infect or otherwise associate with the seed and thus be able to be propagated and sold as a seed line with anti-nematode properties.

### Example 2:

To demonstrate an improved ability of grain-producing cereals such as wheat, or rice to grow in saline soils:
*Step 1:* preferably, plants growing naturally in a saline environment such as a salt marsh or sand dunes (although this is not necessary), are collected together with some "sand/soil/mud" adherent to the roots, and the microorganisms are isolated from roots and stems/foliage either as individual isolates in pure culture or as mixed populations e.g. as a microbial suspension from an aqueous root crush and/or stem/foliar crush, or both, which may be filtered to remove plant debris.
*Step 2:* The microorganisms are added to a plant growth medium containing say ∼100ppm NaCl (wheat) or ∼50ppm NaCl (rice) into which untreated wheat or rice seeds are then planted. Alternatively, the microorganism(s) are mixed into a suitable seed coating material e.g. a gel, and coated onto seeds before being planted into a similar plant growth medium. Alternatively, the seeds are geminated and then exposed to the microorganisms for a short period of say 1 to 24 hours (to maximise the chance that the microbes may form an endophytic or epiphytic association with the germinating plant) and then planted into a similar growth medium. In each of these cases the growing medium may be initially sterile, although this is not essential and further microorganisms applied to the growth medium and/or plant. After a suitable period of plant growth, say a month (but at any desirable time point between germination and seed-harvesting), the plants and/or grain are harvested, adherent soil is gently washed from the roots and relative plant growth of herbage and/or roots determined by dry weight, or image analysis or similar, and/or the grain yield determined, if appropriate for plants left to grow to maturity.
*Step 3:* The plants growing the most, or producing the best seed yield after exposure to saline conditions, are selected and their root and foliar microorganisms isolated and made ready to be added to the seeds and/or the growing medium as individual or combined suspensions, as in step 2. The entire process from step 2 to the end of step 3 can then be repeated iteratively, with or without increasing concentrations of NaCl to increase the selective pressure. After a number of iterations to the point at which there is deemed to be a sufficient improvement in the ability of the wheat or rice plants to resist saline conditions to the degree desired, the microbes in the best-performing plants of the final selection round are isolated and the microbial strains used individually or in a mixture to develop a commercial product that improves the growth of wheat or rice in saline soils (using product development processes and application methodologies similar to that described above in Example 1).

### Example 3:

For specific applications it may be desirable to conduct an initial selection or targeted enrichment process on the microbial population itself, prior to exposure to the target plant, so that the plants finally selected after successive iterations are more likely to be associated with microorganisms with the desirable properties. For example to increase the chance of selecting microorganisms able to withstand environmental extremes e.g. application to bare-rooted pine seedlings prior to planting, during and after which the treated pine seedlings may not be treated with care by the foresters and may dry and/or be exposed to extreme heat and sunlight, or where microorganisms may be coated on to seed which is then planted in an arid soil to await the rains. In such cases as these it may be desirable to pre-select the microbial populations for those that are more likely to withstand such conditions. In the example above the preparation of microorganisms might be pasteurised at 60°C - 80°C for 5-10 minutes thus selecting for the survival of only spore-forming microbes such as bacilli which are able to withstand environmental extremes as well as to associate with plants.

As a further example it may be desirable to pre-select for microbes more likely to provide improved levels of phosphates to the target plant in order to substitute for reduced levels of phosphate fertilisers in the environment of the target plant. In this case it may be desirable to expose the isolated microbial population to an enrichment medium containing phytic acid as the sole source of phosphorus (phytic acid is a model compound for plant phosphates trapped in the soil as phytates, a form of phosphate unavailable to plants but which can be degraded by some microbes to release plant-available phosphorus) and/or microbes could be exposed to an enrichment medium containing hydroxyapatite as the sole source of phosphorus (hydroxyapatite is a model compound for microbes that can release phosphates from rock phosphates). Populations of microorganisms pre-exposed to such selective conditions are likely to be enriched with phosphate-releasing microbes that can then be applied to the plant growth medium as in step 2 of Examples 1 and 2.

In each of the cases above, similar microbial selection procedures may be applied to microorganisms isolated from selected plants at each iteration, although this may not always be required or necessary. It will be appreciated that many microbial selection procedures might similarly be useful and applicable. For example selecting microorganisms to be applied to plant growth medium using media deficient in nitrogen in order to pre-select for microorganisms likely to fix nitrogen and so have the potential to improve plant growth.

### Example 4:

Using environmentally resilient microbes to boost growth of pine seedlings.
a) A *Pinus* variety/species is grown from seed in a variety of soils, including pine forests for 1 - 6 months and microorganisms are isolated from the foliage and roots as in example 1 step 1 and treated to select environmentally-resilient microbes by pasteurisation, as in Example 3.
b) Microbes are applied to growth medium and/or pine seeds or cuttings
c) Seeds/cuttings are grown in growth medium for 3-6 months
d) At 3-6 months the plants are harvested and leaf growth and root growth assessed
e) Desirable plants are selected and microbes isolated and used to inoculate the growth medium and pine seeds/cuttings are planted
f) Steps b to e are repeated iteratively as many times as required
g) Microbes are isolated from pines in the final iteration boost root and/or stem and/or foliar growth

### Example 5:

Use of microbes applied to parts of ryegrass growing above the surface of the ground in order to boost growth:
a) Microorganisms selected from any source, pre-treated or not, pre-selected or not, (as in Example 3) are applied to surfaces of ryegrass plants exposed above the growth medium after growing for any length of time prior to exposure, but preferably within 1-2 months at 22°C in order to shorten the iteration period
b) The plants are grown for a further 1-2 months
c) Foliar growth is assessed and microorganisms from the plants producing the greatest foliar yields are isolated from the foliage.
d) Microorganisms so isolated are reapplied to the surface of ryegrass plants as in step a.
e) Steps a to d are repeated iteratively as many times as required
f) Microbes isolated from foliage of ryegrass plants selected in the final iteration boost root and/or shoot growth

### Example 6:

Many microorganism are antagonistic to one another and the task of finding compatible microbial combinations that exhibit synergistic desirable effects or provide targeted single or multiple benefits or to a plant is onerous. The method of the invention may be used to rapidly identify compatible and/or synergistic combinations/mixtures of microorganisms that provide benefit(s) to a plant using iterative selections of microorganisms in individual culture. For example, microorganisms likely to provide desired benefit(s) are selected from a collection, and applied as mixtures to the plant growth medium. Iterations of plant growth and selection for single or multiple plant attributes are conducted. For example, mixtures of microorganisms may be chosen that could provide either or both disease resistance and improved nitrogen fixation leading to improved plant growth e.g. mixtures of *Rhizobium, Herbaspirillum, Azorhizobium* (nitrogen fixation) coupled with *Bacillus, Pseudomonas* and *Trichoderma* (disease resistance); or improved plant growth in saline conditions coupled with low levels of available phosphorus e.g. *Halomonas, Halobacter* plus *Pantaoea, Enterobacter, Pseudomonas.* Successive rounds of iterative selection for combinations of attributes, as described elsewhere herein, are conducted to select microorganism(s) and microbial mixtures able to multiple plant attributes. As this approach can utilise large numbers of individual microbial cultures covering a broad microbial diversity, it is more likely to identify unexpected microbial synergisms than existing methods, leading to greater than expected plant benefits.

### Example 7:

Use of the invention to identify microorganisms to produce a media or composition (in this example, a "biostimulant fermentation material") that does not contain microorganisms for application to plants e,g, tomatoes to improve their growth:
a) Microorganisms from any source are fermented in two or more (preferably a large number of) mixed cultures using a general fermentation broth that can support the growth of a wide variety of microorganisms, or a range of selective or enrichment media support the growth of microorganisms with an array of separate but desirable properties; for example, media to promote the growth of actinomycetes (antimicrobial metabolites), nitrogen-fixing microorganisms, phosphate-utilising microorganisms and the like. It is envisaged that between 10 - 1000+ individual cultures could be so grown.
b) The microbes in each of these cultures are removed (e.g. by suitable filtration, or by centrifugation) and stored for later use by refrigeration or freezing at -20°C after the addition of cryoprotectants. The separated broths are applied (e.g. by spraying) to individual tomato plants of a desirable size (e.g. 10 cm tall) grown in a suitable growth medium.
c) A period after application e.g. one month, the growth of plants is assessed and the largest plants selected.
d) The microorganisms producing this subset of broths associated with the selected plants are recovered from storage, mixed together and split apart into two or more (preferably a large number of) sub-cultures grown in two or more (preferably a wide range of) different media that are selected using information provided in the selection process, for example it may be evident that the results are skewed towards microorganisms growing in media low in nitrogen, or high in magnesium - in such cases it would be desirable to weight the range of media selected in the second iteration more heavily towards variations in the composition of such media.
e) The process in steps a) to d) is repeated iteratively as many times as is deemed efficacious, with progressive steps refining down to fewer and fewer media and a narrower and narrower diversity of microorganisms until a desirable effect on the growth of tomatoes is achieved with a mixture of microbes that can be identified, grown and stored indefinitely as a standard starting inoculum for the production of a tomato biostimulant product.

It should be appreciated that this method may be applied to any number of a variety of plants. While the method is described in terms of fermenting the microorganisms in a fermentation broth, it should be appreciated that the microorganisms may be contained in alternative media. Further, the plants may be subjected to the broth or media by any appropriate means including direct application or application to the soil, for example. The methods described herein before in respect of applying microorganisms to plants and/or their environment provide further examples. In addition, whilst the product of this method is referred to as a "biostimulant" above, it should be appreciated that the resultant product need not stimulate growth of the plant but may be any media or composition which is of some benefit; for example, is may support growth, health and/or survival of the plant, including provide protection from disease and pests. It is not a requirement of the product that it can support growth and/or health on its own. Finally, reference to "plant(s)" should be taken to include seeds, seedlings, cuttings, and/or propagules thereof.

### Example 8:

To demonstrate an improved ability of a cereal crop such as wheat to withstand an extreme weather condition, such as drought:
a) Preferably, plants growing naturally, or crops, in an arid environment such as a dry savannah, desert, or sand dunes, are collected and microbes isolated as in example 2, although this is not necessary.
b) Microbes are applied to a plant growth medium, preferably sterile, and/or seeds.
c) Seeds grown for a suitable period e.g. 1 month are subjected to a regime of restricted water availability
d) After a suitable period under water restriction the plants surviving are harvested, assessed non-destructively and selected for improved growth of roots and/or foliage.
e) Microorganisms are isolated from the plant tissues of the selected plants and used to inoculate the plant growth medium/plants as in step b).
f) Steps b) to e) are repeated iteratively as many times as required.
g) Microbes are isolated from the best plants in the final iteration and used individually, or in a mixture, to develop a product that improves the resilience of wheat growth under drought conditions.
h) Beneficial changes in plant metabolism following exposure the selected microorganisms that improve the ability of the plant to resist environmental stressors such as heightened salinity, drought or attack by pests and diseases.

### Example 9:

In some cases it may be desirable to select for microbes that are able to impart a desirable plant trait directly from a crop grown in an environment of interest and to use those microbes as a resource to impart the trait to that or other crops. For example to improve the early growth rate of a target crop e.g. maize at 1 month, in either a specific cropping area or in a general cropping region:
a) The largest individual crop plants are selected from multiple fields across a specific cropping area or more general environment of interest one month after planting and microbes are isolated as in Example 2.
b) Microbes from the selected plants are applied to a plant growth medium (preferably sterile), the plants and/or seeds and the plants grown under environmental conditions similar to those experienced in the field for up to one month.
c) One month later maize plants are assessed for the trait non-destructively and selected on the basis of total biomass, root or foliar biomass.
d) Microorganisms are isolated from the plant tissues of the selected plants and used to inoculate the plant growth medium/plants as in step b).
e) Steps b) to e) are repeated iteratively as many times as required.
f) Microbes are isolated from the best plants in the final iteration and used individually, or in a mixture, to develop a product that improves the growth rate of immature maize plants.

### Example 10:

In some applications of the invention it may be desirable to (a) create separate 'lines' of iterative microbial selections for *distinctive plant traits* e.g. pest resistance, nitrogen assimilation, drought resistance, or (b) create separate 'lines' of iterative selections for *the same plant trait* e.g. improvement in biomass production of the target plant, in which the 'lines' of iteratively selected microbial populations originate from target plant microbial populations separated by time or space. After suitable periods of iterative selections, the separately optimised lines can be mixed in order to begin new rounds of iterative selections for joint attributes e.g. pest resistance plus improved nitrogen assimilation, pest resistance plus drought resistance or alternatively for improved growth of a crop plant over an entire growing season. For example, lines of clover-associated microbes separately selected for improved nitrogen assimilation and for nematode resistance can be mixed together as the starting point for a new line of iterative joint selection for the combined attributes. Also, the microbial element of a plant-microbe association may change markedly from germination to maturity e.g. corn (maize). In this case, separate lines of iterative corn selections representing say 2 week, 6 week and 12 week periods of iterative growth and selection are optimised and then mixed to provide microbial populations that combine the best microbial elements of over a 12 week period of crop growth. This new combination can then either be used as the starting point for a new microbial 'line' selected for optimised corn growth to maturity/cob production, or perhaps for combination and development as a commercial product without further optimisation. Alternatively, the three lines may be separately developed as three distinctive products that can be successively applied to a corn crop to 'boost' its growth at different stages of crop development.

### Example 11:

Acquisition of microorganisms from a diverse set of soil samples able to improve the growth of wheat (*Triticum aestivum*) and maize (*Zea mays*)*.*

Diverse microbes associated with the tissues and rhizosphere of two major crops, maize and wheat, were acquired through the selection process described and detailed below.

In brief, plants were grown from seed in a broad range of soil samples of known provenance from the North Island of New Zealand. Microbes from the root tissues and rhizosphere were harvested at maturity and these were then used to inoculate fresh seeds.

After two rounds of directed selection the microbes from the largest plants were isolated and applied individually. There was a significant increase of foliar weight in plants grown with microbes over the controls grown under the same conditions without microbes.

The methodology and results from four examples, namely maize and wheat grown under conditions of low-nitrogen, and wheat and maize grown with insoluble phosphate as the only phosphate source, are given below.

The starting point for all four examples, was a diverse collection of 151 soil samples of known provenance from the North Island of New Zealand. In cases where the soil samples contained roots, these were pulverized and added back to the soil sample. Untreated seeds of wheat and maize were then planted into each sample. Ten replicates were performed for each plant species in 28ml containers filled with soil. Where necessary, the samples were extended by the addition of sterile vermiculite or perlite.

Plants were then grown in the conditions shown in Table 1 with tap water as the only source of moisture. After a suitable period of growth plants were selected on size and the roots and basal stem were harvested by cutting away foliage 1 - 2 cm above the soil line. Excess soil was manually removed and the remaining basal stem and roots were gently washed twice in tap water followed by one rinse in sterile distilled water, leaving small particles of soil attached to the root surfaces. The wet roots of replicate plants from each sample site were combined, placed in sealable plastic bags and crushed. Sterile water (10mls) was added and samples were then filtered through sterile 25um nylon mesh to remove plant material and invertebrate pests.

The resulting microbial suspensions were diluted to an appropriate volume and either enriched for specific microbes or used directly to inoculate surface-sterilized seeds of maize and wheat. Following inoculation with microbes the developing plant and microbe combinations were watered with aqueous fertilizer solutions lacking in either N or soluble P. The general growth conditions are detailed below and specific methodology is given for each example.

**TABLE 1: Standard conditions for all examples**

| **Variable** | **Conditions** |
|---|---|
| Watering | Three times each week to saturation with water or synthetic fertilizer detailed in each section |
| Temperature | Constant 22-24 °C |
| Daylight period | 16 hr followed by 8 hr darkness |
| Seed sterilization | 15 min in 1-2% sodium hypocholorite followed by 30 min quenching in sodium thiosulphate as described by Miche and Balandreau (2001) |
| Volume of soil per replicate | 28 ml |

After two iterations of selection, microbes were isolated from the best individuals and the best-performing sample sets. Microbes isolated to pure culture were subsequently applied to seeds in individual replicated tests to identify those with the ability to enhance the growth of wheat or maize in the absence of nitrogen or soluble phosphate.

### Example 11A:

Improved growth of maize in a nitrogen deficient soil.

Maize plants producing substantial maize growth were selected from the 151 samples for use in experiments to acquire microbes that improve the growth of maize in the absence of nitrogen. The microbial extracts were prepared as detailed above. Surface sterilised maize seeds (*Zea mays,* cultivar Entrée), were placed onto the surface of sterile vermiculite pre-wetted to a suitable level with a sterile N-free liquid fertilizer (CaCl₂ 0.1g/l; MgSO₄.7H₂O 0.12g/l; KH₂PO₄ 0.1g/l; Na₂HPO₄.2H₂O 0.15g/l; FeCl₃ 0.005g/l and a trace mineral solution described by Fahraeus (1957)). Seven replicates were prepared for each treatment, with one seed per replicate. Two ml of extract was pipetted over each seed before it was covered lightly with additional sterile vermiculite. All treatments were watered with sterile N-free fertiliser N+ treatments were watered with the same solution with the addition of 0.355 g/L NH₄NO₃, and sterile distilled water treatments were included to determine the contribution of the media to plant growth. Analysis of maize leaf lengths after 24 days growth revealed differential growth of plants between treatments, with a significant improvement in the ten largest groups of plants over controls containing no added microbes. In the second round of selection the plant growth medium comprised a nitrogen-poor soil containing less than 54kg N/ha (volcanic ash top soil obtained from Paradise Valley, Rotorua, New Zealand). Microbial extracts were prepared from the three largest plants of the 35 treatments producing the longest mean leaf lengths (three longest leaves measured per plant) plus the 15 largest individual plants overall treatments. Ten replicates were inoculated with each of the 50 microbial extracts and 20 replicates were planted for the controls. After 24 days growth maize stems were cut 1cm above the soil and the foliage weighed. The foliar weight was used to select the 8 largest individual plants and the 7 sample sites with the highest average plant weight. Microbial extracts were prepared from the selected plants and groups of plants. Standard volumes (20ul) of these extracts were spread evenly over the surface of global medium (per litre: casein hydrolysate 0.5 g; potato starch 1.0 g; glucose 5.0 g; glycerol 5.0 g; CCY salts (Stewart et al. 1981) 1.0 ml; yeast extract 1.0 g; K₂HPO₄ (10% w/v) 1.0 ml; MgSO₄ 0.1 g; KNO₃ 1.5 g; agar 15.0 g). Colony morphologies were examined under a dissecting microscope and all microbes were assigned to a morphotype. The abundance of each morphotype was assessed by direct microscopic counts of the morphotypes and expressed as cfu/ml. Bacterial and fungal isolates were subsequently identified by 16S rDNA and 18SITS sequencing and were found to comprise a diverse range of microbes from genera known to contain diazotrophs and plant growth promoting microbes as well as some genera not previously associated with plant growth promotion in low nitrogen soils (see Table 2). Sixty-eight isolates cultured from the largest plants were selected from this set based on their abundance and likely utility. These isolates were spread on to ½ TSA agar plates (per litre: casein peptone 15.0 g; soya peptone 5.0 g; NaCl 5.0 g; agar 15.0 g) and grown for 48 hours at 25°C. Plates were then washed using 2 ml sterile distilled water and the number of microbes in each suspension determined by direct counts of colony forming units grown on ½ TSA. Suspensions were diluted to 1x10⁷cfu/ml and 2 ml of this was used to inoculate surface-sterilised maize seeds (n=40 for each treatment). After 26 days growth the foliar matter was cut and weighed. The average foliar weight of plants from the top ten treatments showed a 73% increase over a control treatment with no microbial augmentation. The values shown in Table 2 shows all microbial treatments that resulted in a significant increase (P<0.05, Fisher's LSD) compared with the control.

These results provide evidence that the method for directed selection of microbes described by the present invention is capable of identifying a novel set of microbes able to improve the growth of maize in low-nitrogen conditions.

**TABLE 2: Selected microbes that produced a significant increase in foliar weight when applied to maize grown in nitrogen deficient soil**

| **BDNZ #** | **Mean foliar weight (mg)** | **Putative DNA ID^{a}** |
|---|---|---|
| 54075 | 622 | *Acinetobacter* sp. |
| 54073 | 622 | *Stenotrophomonas maltophilia* |
| 54379 | 600 | *Pantoea dispersa* |
| 54180 | 589 | *Trichosporon* sp. |
| 54385 | 582 | *Rhodococcus erythropolis* |
| 54110 | 581 | *Burkholderia anthina* |
| 54065 | 570 | *Pseudomonas* sp. |
| 54120 | 567 | *Stenotrophomonas maltophilia* |
| 54067 | 567 | *Burkholderia cepacia* |
| 54137 | 564 | *Pantoea agglomerans* |
| 54074 | 563 | *Acinetobacter* sp. |
| 54093 | 563 | *Rhodococcus erythropolis* |
| 54069 | 555 | *Leifsonia aquatica* |
| 54394 | 554 | *Chryseobacterium joostei* |
| 54155 | 554 | *Pantoea ananatis* |
| 54389 | 550 | *Sphingobacterium* sp. |
| 54092 | 549 | Unidentified microbe |
| 54079 | 548 | *Arthrobacter* sp. |
| 54133 | 544 | *Pseudomonas nitroreducens* |
| 54150 | 543 | *Pseudomonas* sp. |
| 54096 | 540 | *Pseudomonas* sp. |
| 54094 | 539 | *Burkholderia phenazinium* |
| 54130 | 537 | *Stenotrophomonas maltophilia* |
| 54154 | 536 | *Leifsonia poae* |
| 54209 | 535 | *Duganella zoogloeoides* |
| 54210 | 529 | *Pseudomonas putida* |
| 54082 | 527 | *Stenotrophomonas maltophilia* |
| 54088 | 524 | *Cryptococcus laurentii* |
| 54104 | 519 | *Trichosporon porosum* |
| N minus control | 397 | No added microbes |

| | | |
|---|---|---|
| ^{a}Identified by 16S/18S rDNA sequencing. | | |

Analysis of Table 2 allowed the construction of consortia based on their relative effects on plant growth and also encompassing microbial diversity. Individual microbes that had been isolated from the best performing sample sets were applied to maize in pair combinations and in combinations of up to five microbes. In another embodiment, the ten microbes associated with the highest mean foliar weight were mixed together in equal ratios and applied to fresh seeds. Microbes were also applied individually to determine any synergistic effects of microbe combinations. The significant effects of individual microbes, alone and in and combination, on the mean weight of plants is shown in Table 3.

**TABLE 3: Microbes and microbial consortia applied to maize grown in a nitrogen-limited soil that produced a significant increase in plant mean foliar weight compared with a microbe-free control.**

| **BDNZ #** | **Mean foliar weight (mg)** | **Putative DNA ID** |
|---|---|---|
| 54079 | 824.8 | *Arthrobacter* sp. |
| 54209 | | *Duganella zoogloeoides* |
| 54075 | | *Acinetobacter* sp. |
| 54379 | | *Pantoea dispersa* |
| 54073 | | *Stenotrophomonas maltophilia* |
| 54065 | 819.6 | *Pseudomonas* sp. |
| 54209 | 800.0 | *Duganella zoogloeoides* |
| 54379 | | *Pantoea dispersa* |
| 54079 | 777.8 | *Arthrobacter* sp. |
| 54073 | | *Stenotrophomonas maltophilia* |
| 54075 | 764.4 | *Acinetobacter* sp. |
| 54073 | | *Stenotrophomonas maltophilia* |
| 54075 | 757.0 | *Acinetobacter* sp. |
| 54379 | | *Pantoea dispersa* |
| 54073 | | *Stenotrophomonas maltophilia* |
| 54209 | 740.4 | *Duganella zoogloeoides* |
| 54093 | | *Rhodococcus erythropolis* |
| 54075 | 738.6 | *Acinetobacter* sp. |
| 54079 | | *Arthrobacter* sp. |
| 54073 | 736.2 | *Stenotrophomonas maltophilia* |
| 54379 | | *Pantoea dispersa* |
| 54110 | 731.8 | *Burkholderia anthina* |
| 54209 | 731.4 | *Duganella zoogloeoides* |
| 54110 | | *Burkholderia anthina* |
| 54209 | 723.1 | *Duganella zoogloeoides* |
| 54079 | 715.5 | *Arthrobacter* sp. |
| 54379 | | *Pantoea dispersa* |
| 54209 | 711.7 | *Duganella zoogloeoides* |
| 54379 | | *Pantoea dispersa* |
| 54110 | | *Burkholderia anthina* |
| 54389 | | *Sphingobacterium* sp. |
| 54079 | 705.5 | *Arthrobacter* sp. |
| 54379 | | *Pantoea dispersa* |
| 54073 | | *Stenotrophomonas maltophilia* |
| 54379 | 700.9 | *Pantoea dispersa* |
| N minus control | 586.4 | No added microbes |

The results of the consortia application to maize grown in nitrogen limited conditions showed clearly that certain combinations of microbes from the best-performing sample sets produced larger plants than individual microbes alone. Although, some microbes appear to increase plant size when alone but not in groups. In particular a strain of Pseudomonas (BDNZ#54065) significantly increased plant size when applied alone but consortia including this strain did not. This may be due to the production of antibacterial compounds. On the other hand a strain of Duganella (BDNZ#54209) increased plant size significantly when applied individually and in combinations containing this microbe also produced significantly larger plants than controls with no added microbes (Table 3). Additionally, the five individual microbes comprising the best treatment in Table 3 above also comprised the individual microbes with the highest frequency of synergistic interactions amongst all of those shown in Table 3. Interestingly the consortia created from microbes isolated from the same site as BDNZ 54209 also produced significantly larger plants than the controls, providing support for the hypothesis that microbial populations have evolved together, either under the selective conditions in the iterations of this example or prior to sample collection.

These results demonstrate that the process of directed selection not only enables the identification of individual microbes that interact with the plant to impart a desired improvement but also enables the ready identification of microbial combinations that unexpectedly perform synergistically to produce an even greater plant response.

### Example 11B:

Improved growth of maize with an insoluble phosphate source using microbes pre-screened for the ability to solubilise phosphate in vitro.

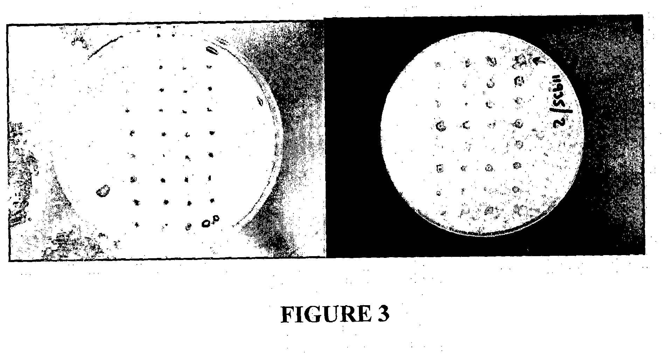

Microbial extracts of maize plants grown in all 151 soil samples were prepared as described above in Example 11. An enrichment step was included for the selection of phosphate solubilising microbes (PSM) associated with maize. 300 µl of microbial extract from the maize tissues and rhizosphere was added to 10 ml Pikovskaya's liquid media (Pikovskaya, 1948) and incubated at room temperature for 5 days with agitation. The presence of PSM was analysed by stabbing 5 µl of liquid culture into Pikovskaya's agar (see Figure 3). Plates were incubated at 28 °C and the clear zones around each well, indicating solubilisation of phosphate, were measured after 24 hrs. Zones were visually scored on the basis of size and clarity on a scale of 0-5 (0=no zone, 5= large clear zone). 60 samples were selected to be carried on further on the basis of these scores.

Cultures from the 60 selected samples were centrifuged for 15 minutes at 4 °C and 20,000 x g in a Sorvall RC 6 centrifuge (Thermo Scientific). The pellet was washed once and then resuspended in a final volume of 60 ml sterile distilled water. *Zea mays* seeds (cultivar Entrée) were soaked in the final suspension for 30 minutes. Seeds (ten replicates for each treatment) were then planted at a depth of 1-2 cm in 28 ml containers filled with low-nutrient volcanic ash soil (Olsen phosphorus 9mg/L, available N 54 kg/ha) that had been saturated with synthetic fertiliser containing insoluble phosphate in the form of tricalcium phosphate (Ca₃(PO₄)₂ or TCP) as the only phosphate source (see Table 4 for composition of fertilisers). Two ml of microbial extract was pipetted on top of each seed and the seeds were then covered with soil.

**TABLE 4: Composition of fertilizers used to saturate the planting substrate in phosphate experiments**

| **BDNZ #** | **Mean foliar weight (mg)** | **Putative DNA ID** |
|---|---|---|
| 54293 | 1026.8 | *Pseudomonas* sp. |
| 54365 | 835.0 | *Pseudomonas* sp. |
| 54299 | 810.0 | *Rhodococcus erythropolis* |
| 54302 | 809.6 | *Pseudomonas veronii* |
| 54364 | 754.2 | *Acinetobacter johnsonii* |
| 54324 | 753.7 | *Acinetobacter* sp. |
| 54374 | 737.7 | *Pantoea ananatis* |
| Ps control | 731.9 | No added microbes |

| | | |
|---|---|---|
| Ps con = soluble phosphate positive control. | | |

Plants were grown for 31 days and watered with ¼ strength Pi fertiliser. After this time the foliage was cut to 1 cm above soil level, weighed and the foliar weight used as an indicator of plant size. Microbial extracts were then prepared from the remaining stem and roots and used to inoculate surface sterilized *Zea mays* seeds in the same low-nutrient soil described above. An aliquot of each sample extract was pooled and autoclaved to determine the contribution of nutrients from microbial extracts to plant growth. Plants were watered with phosphate free fertiliser. After 35 days the foliage was cut and weighed for use in selection of samples for isolation. Microbial extracts were prepared from the five largest individual plants and the pooled plants from the five sample sites with the highest mean weight. Standard volumes (20ul) of these extracts were spread evenly over the surface of global medium. The colony morphology was examined under low magnification and all microbes were initially assigned to a morphotype. The abundance of each morphotype was assessed in relation to the total number of cultured microbes. Isolates were subsequently identified by 16S/18S rDNA sequencing and were found to comprise a diverse range of microbes from genera known to contain PSM but also genera not previously known to contain PSM.

The isolates cultured from the largest plants were spread on ½ TSA and grown overnight. Plates were then washed using 2 ml sterile distilled water and the number of microbes in each suspension was determined by the colony forming units grown on ½ TSA. Suspensions were diluted to 10⁷cfu/ml and 2 ml of this was used to inoculate surface-sterilised maize seeds that had been pre-germinated by incubating in humid conditions for three days at 28 °C. Seeds were planted in the same low-nutrient soil used for microbial selection (n=40 for each treatment). After 22 days the foliar matter was cut and weighed.

Seven treatments performed better than the controls subjected to a soluble phosphate watering regime while all treatments performed better than the controls under the same watering regime with no microbial augmentation.

These results provide evidence that the method for directed selection of microbes described by the present invention is capable of producing a novel set of microbes that significantly improve the growth of maize in conditions where insoluble phosphate is the only phosphate source provided.

### Example 11C:

Improved growth of wheat in a low-phosphorus soil in which insoluble phosphate is the only phosphate source provided.

Microbial extracts were prepared from the 48 most promising treatments of wheat (*Triticumaestivum,* cultivar Raffles) grown for 104 days in the 151 soil samples. The foliage was removed to 1-2 cm above soil level and discarded. Microbial extracts were then prepared from the root, basal stem and rhizosphere as described in Example 11A. Surface-sterilised seeds were planted in ten replicate containers filled with low-nutrient soil saturated with sterile fertiliser solution containing only insoluble tri-calcium phosphate as a phosphate source (see Table 4). Seeds were placed on the surface of the soil and 2 ml of microbial extract was pipetted over them before they were covered lightly with additional soil. After 39 days the foliage was cut and weighed. Microbial extracts were prepared from the 15 treatments with the highest mean weight and the 10 largest individual plants. 20 replicates were planted for each of the 25 treatments. Plants were grown for a period of 29 days then cut and weighed. Microbial extracts were again prepared from the tissues and rhizosphere of the four largest individual plants and the six treatments with the highest mean weight, and spread on an agar medium as described in Example 11A. Isolates were subsequently identified by 16S rDNA sequencing and were found to comprise a diverse range of microbes from genera known to contain phosphate-solubilizing species as well as some genera not previously associated with plant growth promotion in soils with added insoluble phosphate. Eighty-four isolates cultured from the largest plants were selected from this set based on their abundance and likely utility.

Pure cultures of the selected isolates were multiplied on agar plates, harvested and diluted to 1x10⁷cfu/ml after which 2 ml was used to inoculate surface-sterilised wheat seeds (n=30 per treatment) .

After 21 days the foliage was cut and weighed. The average foliar weight of plants from the top ten treatments showed a 37% increase over controls watered with the same insoluble phosphate fertiliser but without added microbes. Statistically significant increases were observed in 56 microbial treatments (Table 5).

These results provide evidence that the method for directed selection of microbes described by the present invention is capable of producing a novel set of microbes that significantly improve the growth of wheat in conditions where insoluble phosphate is the only phosphate source added to a low phosphorus soil.

**TABLE 5: Selected microbes that produced an increase in plant size when applied to maize grown with insoluble phosphate as the sole phosphate source**

| **BDNZ#** | **Mean foliar weight (mg)** | **Putative DNA ID** |
|---|---|---|
| 54499 | 200.4 | *Pantoea agglomerans* |
| 54480 | 197.1 | *Pseudomonas* sp. |
| 54461 | 195.8 | *Arthrobacter nicotinovorans* |
| 54451 | 195.0 | *Bacillus mycoides* |
| 54468 | 194.3 | *Burkholderia gladioli* |
| 54457 | 193.7 | *Janthinobacterium* sp. |
| 54474 | 192.2 | *Pseudomonas veronii* |
| 54460 | 191.5 | *Rhizobium radiobacter* |
| 54472 | 191.0 | *Pedobacter* sp. |
| 54485 | 190.5 | *Pseudomonas* sp. |
| 54517 | 188.9 | *Pseudomonas psychrotolerans* |
| 54470 | 187.5 | *Burkholderia terrae* |
| 54458 | 187.3 | *Mitsuaria chitosanitabida* |
| 54522 | 187.0 | *Bosea thiooxidans* |
| 54481 | 185.3 | *Chryseobacterium joostei* |
| 54463 | 185.0 | *Rhizobium radiobacter* |
| 54459 | 184.3 | *Delftia* sp. |
| 54503 | 184.0 | *Pseudomonas syringae* |
| 54456 | 183.8 | *Janthinobacterium* sp. |
| 54476 | 183.7 | *Pantoea agglomerans* |
| 54482 | 183.4 | *Stenotrophomonas* sp. |
| 54473 | 182.8 | *Microbacterium arabinogalactanolyticum* |
| 54487 | 182.1 | *Herbaspirillum huttiense* |
| 54484 | 181.6 | *Burkholderia gladioli* |
| 54504 | 180.8 | *Bacillus* sp. |
| 54475 | 180.8 | *Herbaspirillum huttiense* |
| 54483 | 179.9 | *Pseudomonas* sp. |
| 54478 | 179.3 | *Chryseobacterium* sp. |
| 54505 | 177.7 | *Sphingomonas koreensis* |
| 54501 | 177.5 | *Pantoea agglomerans* |
| 54464 | 177.1 | *Microbacterium testaceum* |
| 54471 | 176.7 | *Chryseobacterium* sp. |
| 54495 | 176.1 | *Pseudomonas psychrotolerans* |
| 54466 | 174.8 | *Pantoea* sp. |
| 54479 | 174.4 | *Pseudomonas* sp. |
| 54523 | 171.9 | *Rhizobium* sp. |
| 54454 | 170.6 | *Novosphingobium resinovorum* |
| 54500 | 167.3 | *Pseudomonas* sp. |
| 54496 | 167.2 | *Bacillus cereus* |
| 54568 | 166.5 | *Bacillus* sp. |
| 54486 | 166.4 | *Sphingobacterium* sp. |
| 54567 | 165.7 | *Stenotrophomonas* sp. |
| 54462 | 164.6 | *Burkholderia gladioli* |
| 54477 | 164.4 | *Sphingobacterium multivorum* |
| 54467 | 162.3 | *Bacillus cereus* |
| 54548 | 161.8 | *Bacillus* sp. |
| 54543 | 161.0 | *Arthrobacter* sp. |
| 54497 | 160.6 | *Chryseobacterium ureilyticum* |
| 54469 | 158.9 | *Mitsuaria* sp. |
| 54449 | 157.9 | *Enterobacter* sp. |
| 54836 | 157.8 | *Chryptococcus luteolus* |
| 54564 | 156.7 | *Enterobacter ludwigii* |
| 54455 | 156.3 | *Ochrobactrum* sp. |
| 54508 | 156.1 | *Pseudomonas* sp. |
| Pi control | 136.2 | No microbes added |

| | | |
|---|---|---|
| Pi = insoluble phosphate control. | | |

### Example 11D:

Improved growth of wheat in a nitrogen deficient soil.

Microbial extracts were prepared from the 51 most promising treatments of wheat grown for 104 days in the 151 soil samples. The foliage was removed to 1-2 cm above soil level and discarded. Microbial extracts were then prepared from the root, basal stem and rhizosphere as described in Example 11A. Surface-sterilised seeds were planted in ten replicate containers filled with low-nutrient soil and saturated with sterile N-free fertiliser solution described in Example 11A. N+ treatments were watered with the same solution with the addition of 0.355 g/L NH₄NO₃. Sample treatments were watered with N-free solution and a sterile distilled water treatment was included for comparison. Analysis of foliar weights after 40 days revealed differential growth of plants between treatments with 13 treatments producing significantly larger plants than the N-free control without added microbes. Microbial extracts were prepared from the four largest plants (pooled) from the ten treatments producing the highest mean foliar weigh and from the 5 largest individual plants. These 15 extracts were applied to seeds at 20 replicates per treatment for the second round of selection, together with N+, N-free and sterile water treatments. Plants were grown for a period of 29 days then cut and weighed. Microbial extracts were prepared from the three largest individual plants and the four treatments with the highest mean weight, and spread on global agar as described in Example 11A. 79 isolates were subsequently identified by 16S rDNA sequencing and were found to comprise a diverse range of microbes from genera known to contain nitrogen-fixing species as well as genera not previously associated with plant growth promotion in low-nitrogen soils.

42 isolates were chosen for individual application to wheat seeds. Pure cultures of the selected isolates were multiplied on ½ TSA plates, harvested and diluted to 1x10⁷cfu/ml after which 2 ml was used to inoculate surface-sterilised wheat seeds (n=30 per treatment). After 20 days the foliage was cut to 1 cm above soil level and weighed. The average foliar weight of plants from the top ten treatments showed a 40% increase over controls watered with the same nitrogen-free fertiliser but without added microbes. Statistically significant increases were observed in 36 microbial treatments which are shown in Table 6.

**TABLE 6: Identity of microbes applied to wheat that showed significant increase in foliar weight over microbe-free controls**

| **BDNZ#** | **Mean foliar weight (mg)** | **Putative DNA ID** |
|---|---|---|
| 54651 | 226.5 | *Rhodococcus erythropolis* |
| 54578 | 215.8 | *Pseudomonas* sp. |
| 54640 | 202.7 | *Pseudomonas chlororaphis* |
| 54644 | 201.7 | *Pseudomonas* sp. |
| 54577 | 200.1 | *Pseudomonas* sp. |
| 54661 | 196.4 | *Burkholderia phytofirmans* |
| 54579 | 194.6 | *Pantoea agglomerans* |
| 54604 | 194.5 | *Sphingobacterium multivorum* |
| 54582 | 193.6 | *Rhodococcus erythropolis* |
| 54610 | 191.6 | *Stenotrophomonas maltophilia* |
| 54648 | 191.3 | *Pantoea* sp. |
| 54658 | 190.7 | *Burkholderia fungorum* |
| 54660 | 190.7 | *Paenibacillus amylolyticus* |
| 54612 | 189.9 | *Rhodococcus erythropolis* |
| 54659 | 189.8 | *Burkholderia phytofirmans* |
| 54657 | 188.0 | *Janthinobacterium* sp. |
| 54583 | 187.2 | *Arthrobacter* sp. |
| 54600 | 187.2 | *Pantoea ananatis* |
| 54649 | 187.2 | *Pseudomonas* sp. |
| 54620 | 185.9 | *Pantoea agglomerans* |
| 54613 | 184.8 | *Stenotrophomonas* sp. |
| 54619 | 184.2 | *Pseudomonas corrugata* |
| 54624 | 184.2 | *Burkholderia phytofirmans* |
| 54646 | 183.0 | *Alcaligenes faecalis* |
| 54650 | 182.8 | *Comamonas testosteroni* |
| 54599 | 181.0 | *Rhodococcus erythropolis* |
| 54615 | 180.6 | *Curtobacterium* sp. |
| 54617 | 178.3 | *Stenotrophomonas maltophilia* |
| 54588 | 178.1 | *Pseudomonas putida* |
| 54584 | 177.1 | *Sphingomonas* sp. |
| 54587 | 175.2 | *Enterobacter* sp. |
| 54603 | 174.0 | *Burkholderia terrae* |
| 54589 | 173.5 | *Pseudomonas* sp. |
| 54611 | 172.9 | *Arthrobacter nicotinovorans* |
| 54605 | 170.1 | *Microbacterium resistens* |
| 54591 | 169.4 | *Arthrobacter nicotinovorans* |
| N minus control | 145.7 | No added microbes |

### Discussion

The purpose of the experiments outlined in this section was to acquire novel plant-growth promoting bacteria from a collection of 151 soil samples taken from diverse sites. The four examples detailed here provide evidence that the invention is able to rapidly acquire a suite of novel strains of plant-growth promoting microbes without the use of costly and time-consuming molecular characterization. This was achieved by evolving a robust and diverse population of soil microbes to a specific purpose and plant as opposed to performing field experiments on microbes with knowledge of their abilities gained solely from laboratory assays on single cultures. In this way the acquisition of plant growth promoting microbes can be shifted to focus on capturing and identifying the entire culturable diversity of soil before detailed and expensive tests are done on promising individual microbes that fail to survive or compete in soil environments. The numbers of microbes isolated here that show an ability to improve the growth of both wheat and maize point to this invention promising a paradigm shift, both in the way microbes can be acquired and in the way soil populations can be manipulated. The genetic diversity of microbes available on Earth is immense. It is likely that these microbes will have evolved mechanisms to deal with many of the problems facing agriculture today. The invention provides a means to rapidly identify acquire, characterise and subsequently identify such useful microbes.

The methods of the present invention allow for the selection of populations of microorganisms that form associations with different plants and thus confer one or more beneficial properties to the plant, or for the selection of compositions which confirm one or more beneficial properties to the plant. The methods may be conducted with much greater speed and a much-reduced cost of development than conventional techniques (such as selective breeding and genetic engineering) for gaining improvements in plants.

The inventor notes that many of the microorganisms of potential use to plants may be antagonistic to one another and the task of finding compatible microbial combinations that provide targeted multiple benefits to a plant using conventional methods may be onerous. It is envisaged that the methods of the invention are of use in rapidly finding compatible combinations/mixtures of microorganisms that provide multiple benefits to a plant using collections of microorganisms in individual culture.

The potential benefits to and/or improvements in plants that may be gained from the invention include but are not limited to:
a) Protection of foliar, stem, roots and reproductive parts of a plant from or tolerance to attack by invertebrate pests such as insects, nematodes, mites, slugs and snails resulting in improved plant growth;
b) Protection of foliar, stem, roots and reproductive parts of a plant from or tolerance to attack by microorganisms that cause plant diseases, such as Bacteria, Fungi, Protists, Archaea and viruses resulting in improved plant growth.
c) Increased numbers of nodules being induced in the plant. This leads to an increase in the amount of nitrogen that can be "fixed" from the atmosphere by these plants resulting in improved plant growth.
d) Improved plant growth resulting from microbial nitrogen fixation from the atmosphere without the formation of nodules.
e) More efficient release of phosphate more efficiently from the soil resulting in improved plant growth.
f) Changes in the internal microbial ecology of the plant favouring the growth of beneficial microorganisms resulting in improved plant growth.
g) Improvement of a plants ability to capture nutrients and water as well withstand abiotic stresses such as drought or extremes of temperature, light, salinity, or pH or contamination with inorganic or organic compounds of materials toxic to the plant resulting in improved plant growth.

The invention has been described herein, with reference to certain preferred embodiments, in order to enable the reader to practice the invention without undue experimentation. However, a person having ordinary skill in the art will readily recognise that many of the components and parameters may be varied or modified to a certain extent or substituted for known equivalents without departing from the scope of the invention. It should be appreciated that such modifications and equivalents are herein incorporated as if individually set forth. In addition, titles, headings, or the like are provided to enhance the reader's comprehension of this document, and should not be read as limiting the scope of the present invention.

The entire disclosures of all applications, patents and publications, cited above and below, if any, are hereby incorporated by reference. However, the reference to any applications, patents and publications in this specification is not, and should not be taken as, an acknowledgment or any form of suggestion that they constitute valid prior art or form part of the common general knowledge in any country in the world.

Throughout this specification and any claims which follow, unless the context requires otherwise, the words "comprise", "comprising" and the like, are to be construed in an inclusive sense as opposed to an exclusive sense, that is to say, in the sense of "including, but not limited to".

### BIBLIOGRAPHY

Pikovskaya RI (1948). Mobilization of phosphorus in soil connection with the vital activity of some microbial species. Microbiologiya 17:362-370
Miche, L and Balandreau, J (2001). Effects of rice seed surface sterilisation with hypochlorite on inoculated Burkholderiavietamiensis. Appl. Environ. Microbiol.67(7): p3046-3052
Fahraeus, G. (1957). J. Gen Microbiol. 16: 374-381

The invention further includes the subject matter of the paragraphs of PCT/NZ2012/000041 from which this application is derived, the content of which is reproduced below as numbered paragraphs (paras).
1. A method for the selection of one or more microorganisms capable of imparting one or more beneficial property to a plant, the method comprising at least the steps of:
   a) subjecting one or more plant (including seeds, seedlings, cuttings, and/or propagules thereof) to a growth medium in the presence of one or more microorganisms;
   b) applying one or more selective pressure during step a);
   c) selecting one or more plant following step b); and,
   d) isolating one or more microorganisms from said one or more plant selected in step c).
2. A method according to paragraph 1, wherein the step of subjecting one or more plant to a growth medium involves growing or multiplying the plant.
3. A method according to paragraph 1 or 2, wherein one selective pressure is applied in step b).
4. A method according to any one of paragraphs 1 to 3, wherein the selective pressure is biotic.
5. A method according to paragraph 4, wherein the selective biotic pressure includes exposure to one or more organisms that are detrimental to the plant.
6. A method according to paragraph 5, wherein the organisms are selected from fungi, bacteria, viruses, insects, mites and nematodes.
7. A method according to any one of paragraphs 1 to 3, wherein the selective pressure is abiotic.
8. A method according to paragraph 7, wherein the abiotic selective pressure is selected from exposure to or changes in the level of; salt concentration, temperature, pH, water, minerals, organic nutrients, inorganic nutrients, organic toxins, inorganic toxins, and metals.
9. A method according to any one of paragraphs 1 to 8, wherein the selective pressure is applied during substantially the whole time during which the one or more plant is subjected to the growth medium and one or more microorganisms.
10. A method according to any one of paragraphs 1 to 8, wherein the selective pressure is applied during substantially the whole growth period of the one or more plant.
11. A method according to any one of paragraphs 1 to 8, wherein the selective pressure is applied at a discrete time point.
12. A method according to any one of paragraphs 1 to 11, wherein the one or more plant is selected on the basis of one or more phenotypic trait.
13. A method according to any one of paragraphs 1 to 12, wherein the one or more microorganisms are isolated from the root, stem and/or foliar (including reproductive) tissue, or whole plant tissue of the one or more plants selected.
14. A method according to any one of paragraphs 1 to 13, wherein the one or more microorganisms are isolated from the one or more plants any time after germination.
15. A method according to any one of paragraphs 1 to 13, where two or more microorganisms are isolated in step d), the method further comprises the steps of separating the two or more microorganisms into individual isolates, selecting two or more individual isolates, and then combining the selected two or more isolates.
15. A method according to any one of paragraphs 1 to 14, wherein the method further comprises repeating steps a) to d) one or more times, wherein the one or more microorganisms isolated in step d) are used in step a) of the successive repeat.
16. A method according to paragraph 15, wherein the one or more selective pressure is applied in successive repeats of steps a) to d) is different.
17. A method according to paragraphs 15 or 16, wherein the one or more selective pressure applied in successive repeats of steps a) to d) is the same.
18. A method according to any one of paragraphs 1 to 17, wherein prior to step a) the method comprises subjecting the one or more plant (including seeds, seedlings, cuttings, and/or propagules thereof) to a growth medium in the presence of one or more microorganisms, and after a desired period, isolating one or more microorganisms from said one or more plant.
19. A method according to paragraph 18, wherein the one or more microorganisms isolated from the one or more plant, are used in step a) of the process.
20. A method according to any one of paragraphs 1 to 19, wherein two or more methods are performed separately and the one or more microorganisms isolated in step d) of each separate method are combined.
21. A method according to paragraph 20, wherein the combined microorganisms are used in step a) of the process.
22. A method for the selection of one or more microorganisms capable of imparting one or more beneficial property to a plant or for the selection of a plant harbouring one or more microorganisms capable of imparting one or more beneficial property to a plant, the method comprising at least the steps of:
   a) subjecting one or more plant (including seeds, seedlings, cuttings, and/or propagules thereof) to a growth medium in the presence of one or more microorganisms;
   b) applying one or more selective pressure during step a); and,
   c) selecting one or more plant following step b).
23. A method for assisting in the improvement of one or more plants, comprising arranging for the evaluation of said plant(s) in the presence of one or more microorganism and/or one or more composition.
24. A method according to paragraph 23, wherein the plant(s) are for growing in a first region.
25. A method according to paragraph 23 wherein the microorganism(s) may or may not (or at least to a significant extent) be present in the first region.
26. A method according to any one of paragraphs 23 to 25 wherein the evaluation is performed in a second region.
27. A method according to any one of paragraphs 23 to 26 wherein, the step of arranging comprises arranging for one or more of:
   - receipt or transmission of an identity of one or more plants or plant types to be evaluated;
   - receipt or transmission of plant material from one or more plants or plant types to be evaluated;
   - identification and / or selection of the microorganism(s) and/or composition(s);
   - acquisition of the microorganism(s) and/or composition(s); and
   - associating the microorganism(s) and/or composition(s) with the plant material.
28. A method according to any one of paragraphs 23 to 27, wherein the method comprises evaluating said plant(s) in the presence of said microorganism(s) and/or composition(s).
29. A method according to any one of paragraphs 23 to 28, wherein the method comprises arranging for said evaluation of said plant(s) in the presence of said microorganism(s) and/or composition(s).
30. A method according to any one of paragraphs 23 to 28, wherein the evaluating comprises performing one or more of the methods of paragraphs 1 to 22 or 45 or 46.
31. A method according to any one of paragraphs 23 to 30 wherein the steps are performed by a single entity.
32. A method according to any one of paragraphs 23 to 30 wherein the steps are performed by at least two parties, a first which makes a request and a second which actions the request.
33. A method according to any one of paragraphs 23 to 32, further comprising one or more of:
   - receiving or sending one or more microorganisms (or at least the identity thereof) and/or composition(s) to the first region, including in combination with plant material; and
   - growing said plant(s) or other plants (preferably having similar properties) in the first region in the presence of said microorganism(s) and/or composition(s).
34. A method according to any one of paragraphs 23 to 33, wherein a first party:
   - identifies a need for an improvement in a plant(s);
   - sends the identity thereof and / or relevant plant material to a second party together with any relevant information, and
   - receives plant material and / or one or more microorganisms and / or the identities thereof and/or composition(s).
35. A method of paragraph 34, wherein the step of receiving is performed following or as a result of an assessment of plant/microorganism and/or plant/composition associations.
36. A method according to any one of paragraphs 23 to 35 wherein a second party:
   - receives an identity of a plant(s) and / or relevant plant material from a first party together with any relevant information, and
   - sends plant material and / or one or more microorganisms and / or the identities thereof and/or composition(s) to the first party.
37. A method according to paragraph 36, wherein the step of sending is performed following or as a result of an assessment of plant / microorganism and/or plant/composition associations.
38. A system for implementing the method of any one of paragraphs 23 to 37.
39. A system according to paragraph 38, including one or more of:
   - means for receiving or transmitting an identity of one or more plants or plant types to be evaluated;
   - means for receiving or transmitting plant material from one or more plants or plant types to be evaluated;
   - means for identifying and / or selecting microorganism(s) and/or composition(s);
   - means for acquiring the microorganism(s) and/or composition(s);
   - means for associating the microorganism(s) and/or composition(s) with the plant material;
   - means for evaluating said plant(s) in the presence of said microorganism(s) and/or composition(s);
   - means for receiving or sending one or more microorganisms (or at least the identity thereof) and/or composition(s) to the first region, including in combination with plant material; and
   - means for growing said plant(s) or other plants (preferably having similar properties) in the first region in the presence of said microorganism(s) and/or composition(s).
40. A system according to paragraph 38 or 39 wherein the system is embodied by a facility configured to transmit request(s) for an improvement in a plant(s) and subsequently to receive plant material and / or one or more microorganisms and / or the identities thereof and/or composition(s).
41. A system according to paragraph 38 or 39 wherein the system is embodied by a facility configured to receive an identity of a plant(s) and / or relevant plant material from together with any relevant information; and send plant material and / or one or more microorganisms and / or the identities thereof and/or composition(s), preferably following or as a result of an assessment of plant / microorganism and/or plant/composition associations.
42. A microorganism isolated by a method according to any one of paragraphs 1 to 21.
43. A plant obtained by a method of paragraph 22.
44. A method for the production of a composition to support plant growth and/or health, the method comprising the steps of a method according to any one of paragraphs 1 to 21 and the additional step of combining the one or more microorganisms isolated from said one or more plant with one or more additional ingredients.
45. A composition comprising one or more microorganism according to paragraph 42.
46. A combination of two or more microorganism isolated by the method according to any one of paragraphs 1 to 21.
47. A method for the selection of a composition which is capable of imparting one or more beneficial property to a plant, the method comprising at least the steps of:
   a) culturing one or more microorganism in one or more media;
   b) separating the one or more microorganism from the one or more media after a period of time to provide one or more composition;
   c) subjecting one or more plant (including seeds, seedlings, cuttings, and/or propagules thereof) to the one or more composition;
   d) selecting one or more composition if it is observed to impart one or more beneficial property to the one or more plants.
48. A method for the selection of one or more microorganisms which are capable of producing a composition which is capable of imparting one or more beneficial property to a plant, the method comprising at least the steps of:
   a) culturing one or more microorganism in one or more media;
   b) separating the one or more microorganism from the one or more media after a period of time to provide one or more composition;
   c) subjecting one or more plant (including seeds, seedlings, cuttings, and/or propagules thereof) to the one or more composition;
   d) selecting the one or more microorganisms associated with one or more composition observed to impart one or more beneficial property to the one or more plants.
49. A composition selected by a method according to paragraph 47.
50. A microorganism selected by a method according to paragraph 48.
51. Use of one or more microorganism identified by a method of any one of paragraphs 1 to 21 for imparting a beneficial property to one or more plant.
52. Use as described in paragraph 51 wherein the one or more microorganism is chosen from the group consisting of the microorganisms listed in tables 2, 3, 5, 6 and 7.
53. Use as described in paragraph 52 wherein the one or more microorganisms is chosen from the group consisting of the microorganisms listed in tables 2, 3, and 7 and the beneficial property is improved growth in nitrogen deficient or nitrogen limited growth media.
54. Use as described in paragraph 52 wherein the one or more microorganisms is chosen from the group consisting of the microorganisms listed in tables 5 and 6 and the beneficial property is improved growth in growth media in which phosphate is present substantially only in insoluble form.
55. Use as described in paragraph 53 wherein the microorganism is Duganella sp.
56. Use as described in paragraph 53 wherein a combination of Arthrobacta sp, Duganella sp, Acinetobacter sp, Panteoa sp, and Stenotrophomonas sp is used.

## Claims

1. A method for the selection of one or more bacteria capable of imparting one or more beneficial property to a plant, the method comprising at least the steps of:
a) subjecting one or more plant to a growth medium in the presence of a mixed population of bacteria;
b) applying one or more selective pressure during step a);
c) selecting one or more plant following step b), based on one or more plant genotypic or phenotypic trait;
d) isolating a bacterial population or obtaining a bacterial population in crude form from said one or more plant and/or plant rhizosphere of said one or more plant selected in step c); wherein,
steps a) to d) are repeated one or more times, wherein the bacterial population isolated or obtained in step d) is used in step a) of any successive repeat.

2. A method according to claim 1, wherein the step of subjecting one or more plant to a growth medium involves growing or multiplying the plant.

3. A method according to claim 1 or claim 2, wherein the one or more selective pressure is biotic or abiotic.

4. A method according to any one of claims 1 to 3, wherein the one or more selective pressure is applied: during substantially the whole time during which the one or more plant is subjected to the growth medium and the mixed population of bacteria; during substantially the whole growth period of the one or more plant; and/or at a discrete time point.

5. A method according to any one of claims 1 to 4, wherein the selection in step c) is based on one or more phenotypic trait.

6. A method according to any one of claims 1 to 5, wherein the bacterial population is isolated or obtained from the root, stem and/or foliar (including reproductive) tissue, or whole plant tissue of the one or more plants selected.

7. A method according to any one of claims 1 to 6, wherein the bacterial population is isolated or obtained from the one or more plants any time after germination.

8. A method according to any one of claims 1 to 7, wherein the method further comprises the steps of separating bacterial population into individual isolates, selecting two or more individual isolates, and then combining the selected two or more isolates.

9. A method according to any one of claims 1 to 8, wherein the one or more selective pressure is applied in successive repeats of steps a) to d) is: different; or, the same.

10. A method according to any one of claims 1 to 9, wherein two or more methods are performed separately and the bacterial population isolated or obtained in step d) of each separate method are combined.

11. A method according to claim 10, wherein the combined bacterial populations are used in step a) of any successive repeat of steps a) to d) of the method of any one of claims 1 to 10.

12. A method according to any one of claims 1 to 11, wherein the one or more plant is a seed, seedling, cutting, propagule and/or any other plant material or tissue capable of growing.

13. A method as claimed in any one of claims 1 to 12 wherein the method further comprises the additional step of combining the one or more bacteria selected with one or more additional ingredients for the production of a composition to support plant growth and/or health.
